(19) Europäisches Patentamt
European Patent Office
Office européen des brevets

(11) **EP 2 674 912 B1**

(12) **EUROPEAN PATENT SPECIFICATION**

(45) Date of publication and mention of the grant of the patent:
**21.11.2018 Bulletin 2018/47**

(51) Int Cl.:
***G06T 7/00*** *(2017.01)*

(21) Application number: **13169008.3**

(22) Date of filing: **23.05.2013**

(54) **Thoracic diagnosis assistance information generation method, thoracic diagnosis assistance system, and dynamic state image processing apparatus**

Verfahren zur Erzeugung von Thoraxdiagnosehilfsinformationen, Thoraxdiagnosehilfssystem und dynamische Zustandsabbildungsverarbeitungsvorrichtung

Procédé de génération d'informations d'assistance au diagnostic thoracique, système d'assistance au diagnostic thoracique et appareil de traitement d'image à l'état dynamique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2012 JP 2012131594**

(43) Date of publication of application:
**18.12.2013 Bulletin 2013/51**

(73) Proprietor: **Konica Minolta, Inc.**
**Tokyo 100-7015 (JP)**

(72) Inventors:
• **Noji, Sho**
**Tokyo, Tokyo 100-7015 (JP)**

• **Muraoka, Shintaro**
**Tokyo, Tokyo 100-7015 (JP)**

(74) Representative: **Higgs, Jonathan et al**
**Urquhart-Dykes & Lord LLP**
**Altius House**
**1 North Fourth Street**
**Milton Keynes, MK9 1NE (GB)**

(56) References cited:
**WO-A1-2012/026145     WO-A1-2012/026146**
**US-A1- 2003 103 663     US-A1- 2012 130 238**

**Description**

BACKGROUND

Field of the Invention

The present invention relates to a thoracic diagnosis assistance information generation method, a thoracic diagnosis assistance system, and a dynamic image processing apparatus.

Description of Related Art

Instead of stationary image capturing and diagnosis by thoracic radiation using a conventional film/screen or photostimulable phosphor plate, there is an approach to capture a thoracic dynamic image using a semiconductor image sensor such as an FPD (flat panel detector), etc., to be applied to diagnosis.

Specifically, fast responsiveness of reading and deleting of image data of the semiconductor image sensor is used and pulsed radiation is successively emitted from a radiation source to match with the timing of reading and deleting by the semiconductor image sensor to perform capturing a plurality of times in one second. With this, a thoracic dynamic state is captured. A string of plurality of images obtained by capturing are sequentially displayed and it is possible for a physician to observe a string of movement of a chest portion according to breath movement, heartbeat, etc.

Various techniques are proposed to generate and provide information useful in diagnosis based on a thoracic dynamic image. For example, the inventor of the present invention claims a technique in which a lung field region is extracted from a dynamic image of a chest portion and divided into small regions, air velocity ratio (maximum air velocity of inspiration/ maximum air velocity of expiration) is calculated for each small region and a histogram is displayed in order to provide diagnosis assistance information when a physician diagnoses whether the captured lung field is normal, has an obstructive disease or has a mixed disease (see Patent Document 1: WO 2012/026146).

However, among ventilation diseases of the lung, there are diseases where a difference cannot be seen between the inspiration air velocity and the expiration air velocity.

FIG. 11 shows an inspiration maximum air velocity image 34a, an expiration maximum air velocity image 34b, an air velocity ratio image 34c, and an air velocity ratio histogram 34d of a normal lung field.

FIG. 12 shows an inspiration maximum air velocity image 34a, an expiration maximum air velocity image 34b, an air velocity ratio image 34c, and an air velocity ratio histogram 34d of a lung field classified as GOLD 3 as a result of the spirometric examination.

FIG. 13 shows an inspiration maximum air velocity image 34a, an expiration maximum air velocity image 34b, an air velocity ratio image 34c, and an air velocity ratio histogram 34d of a lung field classified as GOLD 4 as a result of the spirometric examination.

The maximum air velocity image is an image dividing the lung field region by chroma (luminance) according to maximum air velocity. The air velocity ratio image is an image dividing the lung field region by chroma (luminance) according to the air velocity ratio.

Here, GOLD 1 to 4 are indexes showing degree of limit of air flow classified by spirometric examination. The larger the numeric value of GOLD is, the heavier the limit of air flow is.

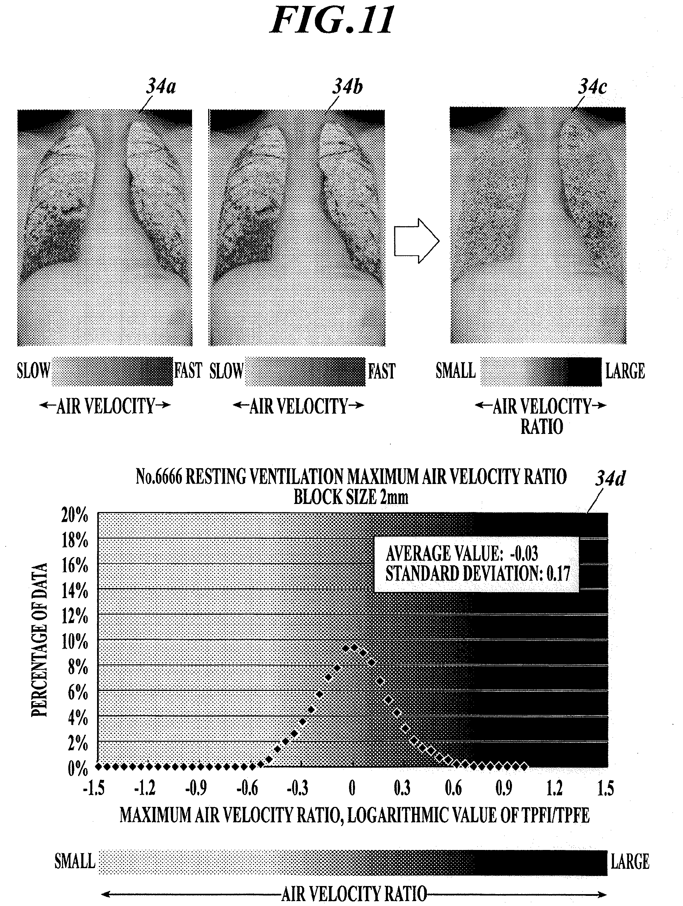

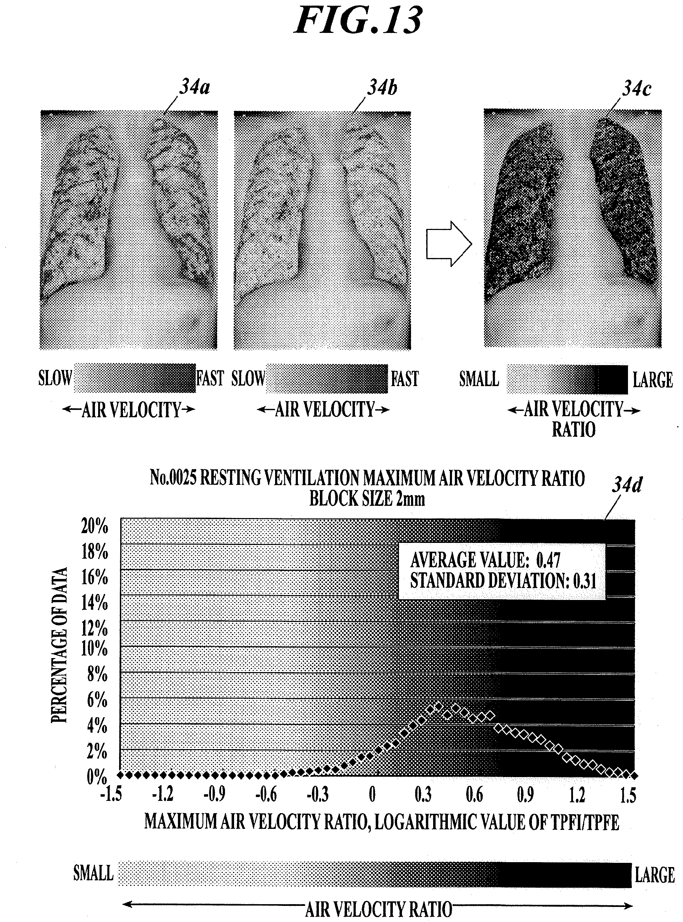

According to the inspiration maximum air velocity image 34a, the expiration maximum air velocity image 34b, and the air velocity ratio image 34c of the lung field as shown in FIG. 13, it is clear that inspiration air velocity > expiration air velocity and it is clear that the shape of the air velocity ratio histogram is different from that of the normal lung field shown in FIG. 11. Such disease can be easily determined by the technique described in patent document 1.

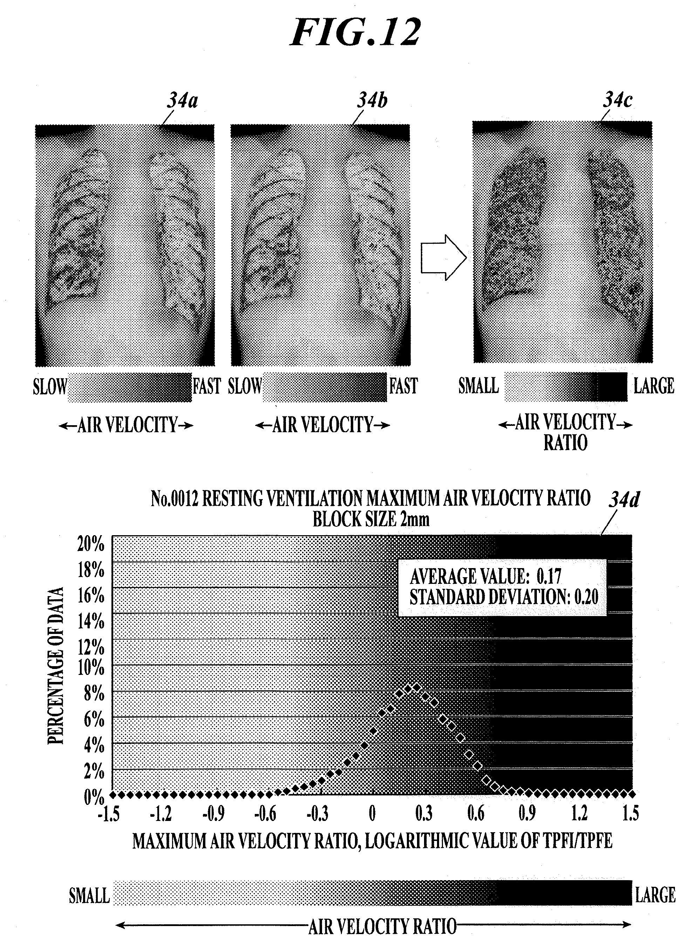

The lung field shown in FIG. 12 is a lung field classified as GOLD 3 as a result of spirometric examination. The difference between the expiration air velocity and the inspiration air velocity cannot be clearly seen. Moreover, the shape of the air velocity ratio histogram is not clearly different from that of the normal lung field shown in FIG. 11. There is a problem that such disease is difficult to detect with the technique described in patent document 1.

SUMMARY

The present invention has been made in consideration of the above problems, and it is one of main objects to generate diagnosis assistance information so that a physician is able to easily acknowledge a ventilation disease in which a difference between the inspiration air velocity and the expiration air velocity cannot be seen.

In order to achieve at least one of the above-described objects, according to an aspect of the present invention, there is provided a thoracic diagnosis assistance information generation method including: capturing a dynamic state of a chest portion including at least one breathing cycle using a detector with detecting elements aligned two dimensionally to generate a plurality of successive frame images; extracting a lung field region from one frame image among the plurality of frame images generated in the capturing; calculating air velocity by dividing the lung field region extracted in the extracting into a plurality of small regions, corresponding the small regions among the plurality of frame images, performing analysis for each corresponded small region, and calculating a feature amount showing air velocity for each small region; and calculating air velocity distribution by dividing the lung field region into a plurality of block regions in a trunk axis direction, calculating a feature amount showing air velocity of each block region based on a feature amount showing the air velocity of the plurality of small regions included in the divided block regions, and calculating a feature amount showing air velocity distribution of the lung field region in the trunk axis direction based on the calculated feature amount showing the air velocity of each block region, wherein the air velocity distribution calculating section is configured to read from a storage section a template showing air velocity distribution of a normal lung field and calculate, as a feature amount showing air velocity distribution of the lung field region in the trunk axis direction, a degree of match of air velocity distribution between the lung field region and a lung field region in the template, based on a feature amount showing air velocity of the plurality of block regions and a feature amount showing air velocity in each corresponding region in the template, and wherein the calculating (S28) of the air velocity distribution includes calculating cross-correlation coefficient (S39) of the lung field region and the lung field region of the template as the degree of match of the air velocity distribution between the lung field region and the lung field region of the template.

Preferably, in the thoracic diagnosis assistance information generation method, calculating the air velocity distribution includes dividing each of a left lung field region and a right lung field region into a plurality of block regions in the trunk axis direction, and calculating a feature amount showing distribution of air velocity for each of the left lung field region and the right lung field region in the trunk axis direction. Preferably, in the thoracic diagnosis assistance information generation method, calculating the air velocity distribution includes dividing the lung field region into three or more block regions in a trunk axis direction. Preferably, in the thoracic diagnosis assistance information generation method, calculating the air velocity distribution includes dividing the lung field region into a plurality of block regions in the trunk axis direction and a direction substantially orthogonal to the trunk axis direction. Preferably, in the thoracic diagnosis assistance information generation method, the template is created based on air velocity distribution of a plurality of normal lung fields. In the thoracic diagnosis assistance information generation method, the calculating of the air velocity distribution includes calculating cross-correlation coefficient of the lung field region and the lung field region of the template as the degree of match of the air velocity distribution between the lung field region and the lung field region of the template.

According to an aspect of the present invention, there is provided a thoracic diagnosis assistance system including: a capturing section which captures a dynamic state of a chest portion including at least one breathing cycle to generate a plurality of successive frame images; an extracting section which extracts a lung field region from one frame image among the plurality of frame images generated by the capturing section; an air velocity calculating section which divides the lung field region extracted by the extracting section into a plurality of small regions, corresponds the small regions among the plurality of frame images, performs analysis for each corresponded small region, and calculates a feature amount showing air velocity for each small region; and an air velocity distribution calculating section which divides the lung field region into a plurality of block regions in a trunk axis direction, calculates a feature amount showing air velocity of each block region based on a feature amount showing the air velocity of the plurality of small regions included in the divided block regions, and calculates a feature amount showing air velocity distribution of the lung field region in the trunk axis direction based on the calculated feature amount showing the air velocity of each block region, wherein the air velocity distribution calculating section is configured to read from a storage section a template showing air velocity distribution of a normal lung field and calculate, as a feature amount showing air velocity distribution of the lung field region in the trunk axis direction, a degree of match of air velocity distribution between the lung field region and a lung field region in the template, based on a feature amount showing air velocity of the plurality of block regions and a feature amount showing air velocity in each corresponding region in the template, wherein the air velocity distribution calculating section (3) is configured to calculate cross-correlation coefficient (S39) of the lung field region and the lung field region of the template as the degree of match of the air velocity distribution between the lung field region and the lung field region of the template.

According to an aspect of the present invention, there is provided a dynamic state image processing apparatus including: an extracting section which extracts a lung field region from one frame image among the plurality of frame images obtained by capturing a dynamic state of a chest portion including at least one breathing cycle; an air velocity calculating section which divides the lung field region extracted by the extracting section into a plurality of small regions, corresponds the small regions among the plurality of frame images, performs analysis for each corresponded small region, and calculates a feature amount showing air velocity for each small region; and an air velocity distribution calculating section which divides the lung field region into a plurality of block regions in a trunk axis direction, calculates a feature amount showing air velocity of each block region based on a feature amount showing the air velocity of the plurality of small regions included in the divided block regions, and calculates a feature amount showing air velocity distribution of the lung field region in the trunk axis direction based on the calculated feature amount showing the air velocity of each block region, wherein the air velocity distribution calculating section is configured to read from a storage

3

section a template showing air velocity distribution of a normal lung field and calculate, as a feature amount showing air velocity distribution of the lung field region in the trunk axis direction, a degree of match of air velocity distribution between the lung field region and a lung field region in the template, based on a feature amount showing air velocity of the plurality of block regions and a feature amount showing air velocity in each corresponding region in the template, wherein the air velocity distribution calculating section (3) is configured to calculate cross-correlation coefficient (S39) of the lung field region and the lung field region of the template as the degree of match of the air velocity distribution between the lung field region and the lung field region of the template.

According to the present invention, it is possible to generate diagnosis assistance information so that the physician can easily acknowledge a ventilation disease in which a difference between the inspiration air velocity and the expiration air velocity cannot be seen.

The present invention will become more fully understood from the detailed description given hereinbelow and the appended drawings, and thus are not intended to define the limits of the present invention, and wherein;

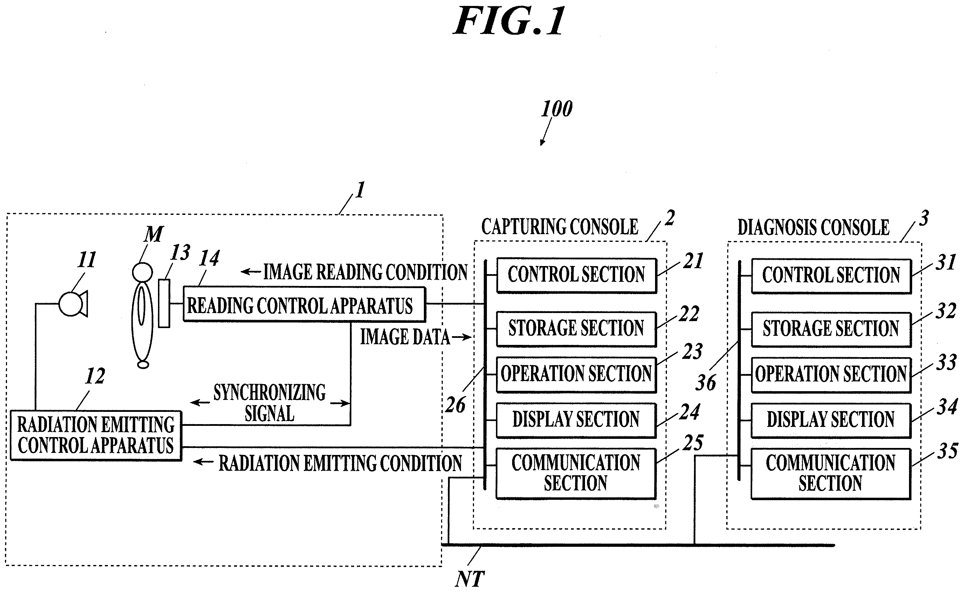

FIG. 1 is a diagram showing an entire configuration of a thoracic diagnosis assistance system of an embodiment of the present invention;

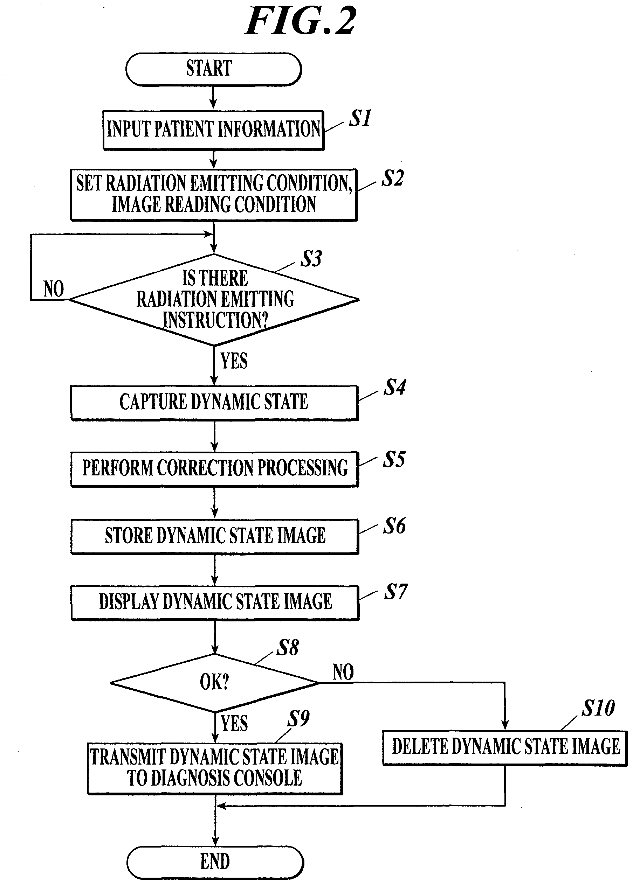

FIG. 2 is a flowchart showing capturing control processing performed by the control section of the capturing console shown in FIG. 1;

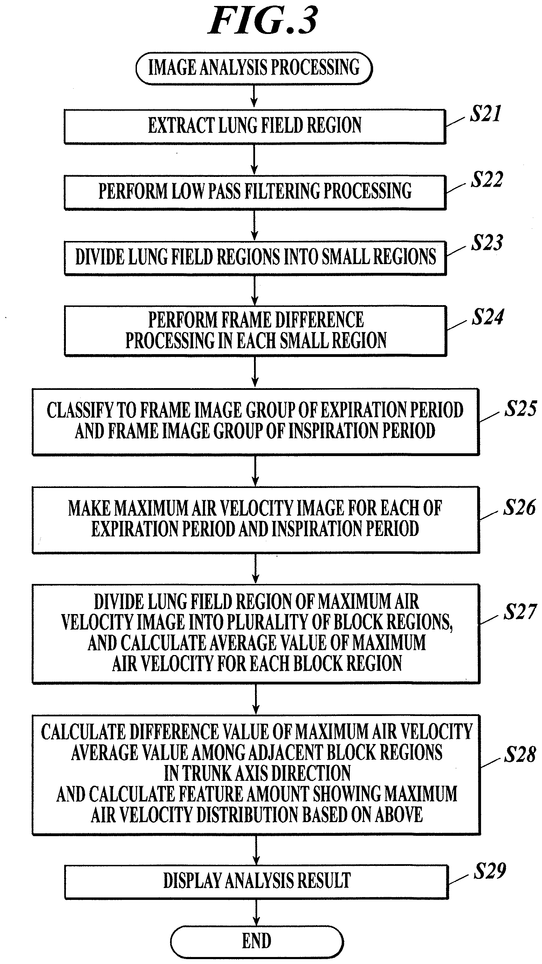

FIG. 3 is a flowchart showing image analysis processing performed by the control section of the diagnosis console shown in FIG. 1;

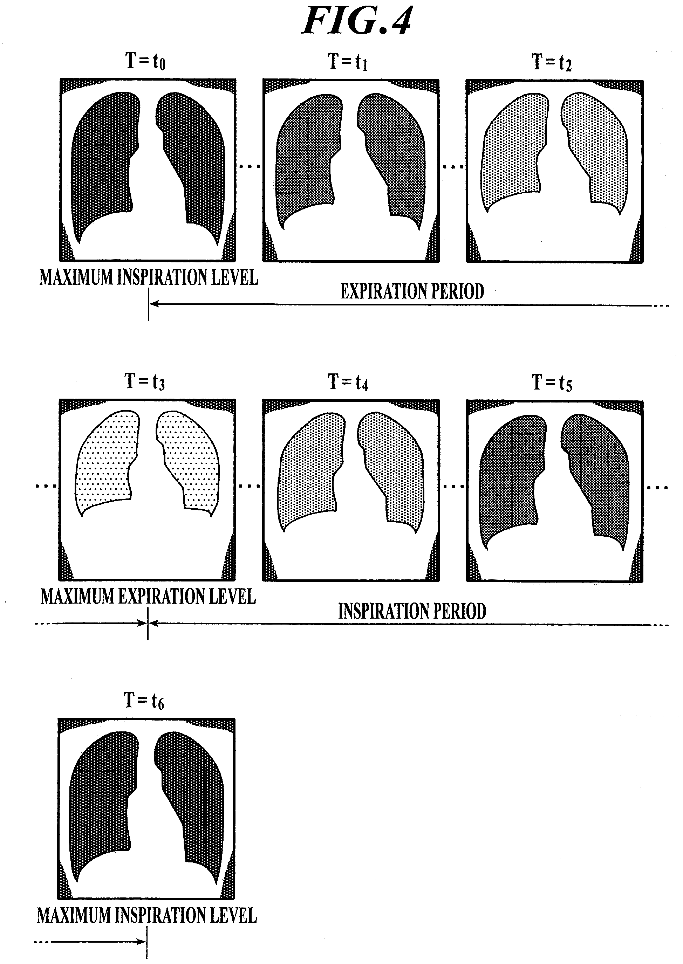

FIG. 4 is a diagram showing a frame image of a plurality of time phases T (T= t0 to t6) captured in one breathing cycle (deep breathing);

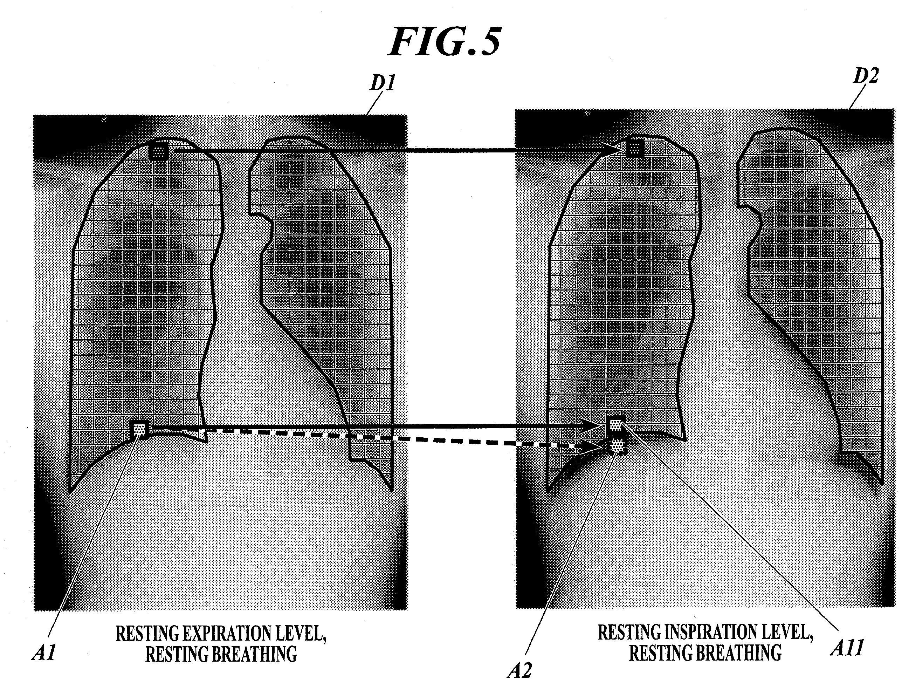

FIG. 5 is a diagram showing a change of a position of a region drawing the same portion of a lung field in resting expiration level and resting inspiration level;

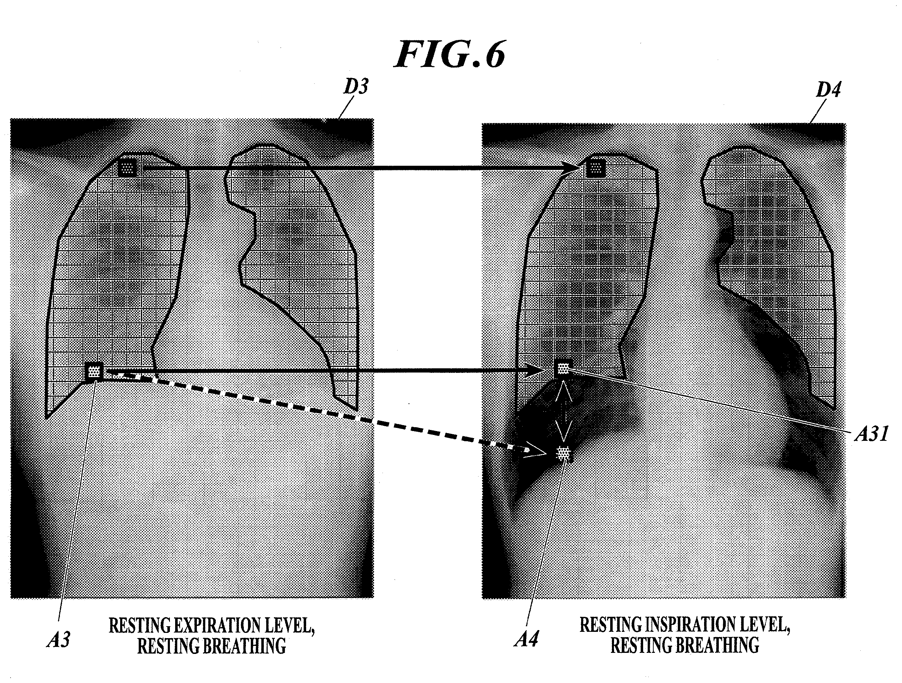

FIG. 6 is a diagram showing a change of a position of a region drawing the same portion of a lung field in maximum expiration level and maximum inspiration level;

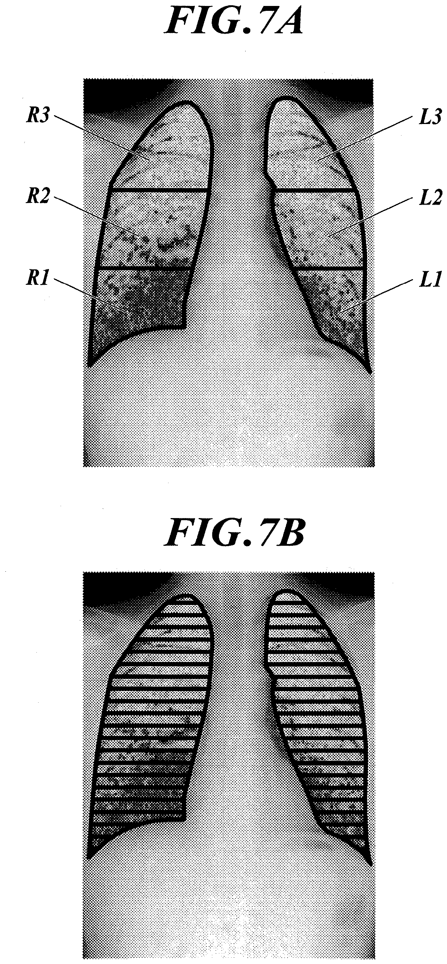

FIG. 7A is a diagram showing an example of dividing each of left and right lung field regions into three block regions;

FIG. 7B is a diagram showing an example of dividing each of left and right lung field regions into twenty block regions;

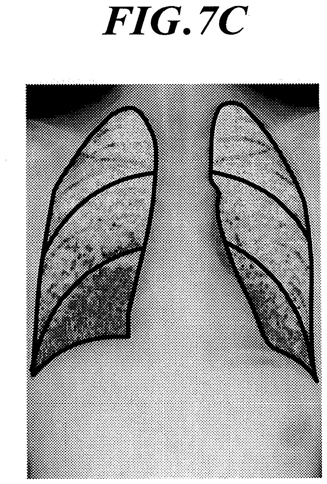

FIG. 7C is a diagram showing an example of dividing each of left and right lung field regions into three block regions according to clinical practice;

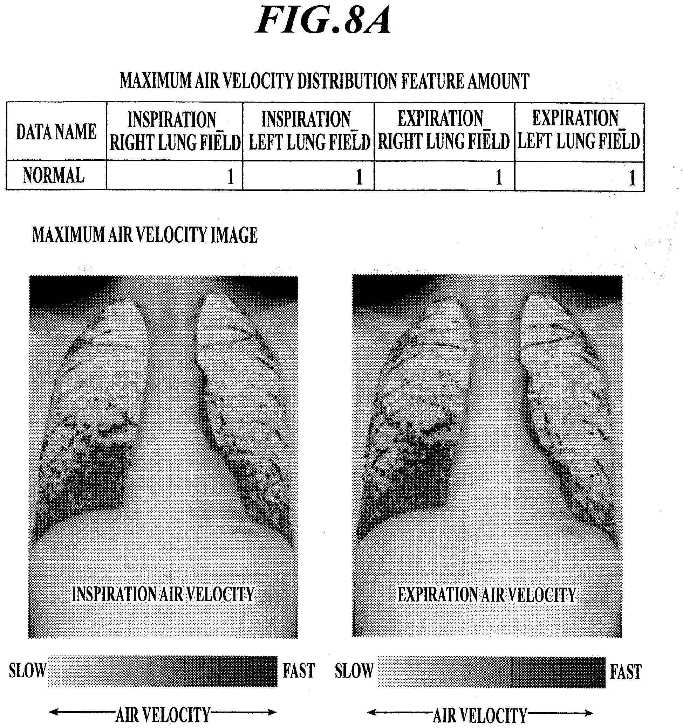

FIG. 8A is a diagram showing a display example of an analysis result displaying a maximum air velocity distribution feature amount and a maximum air velocity image for each small region;

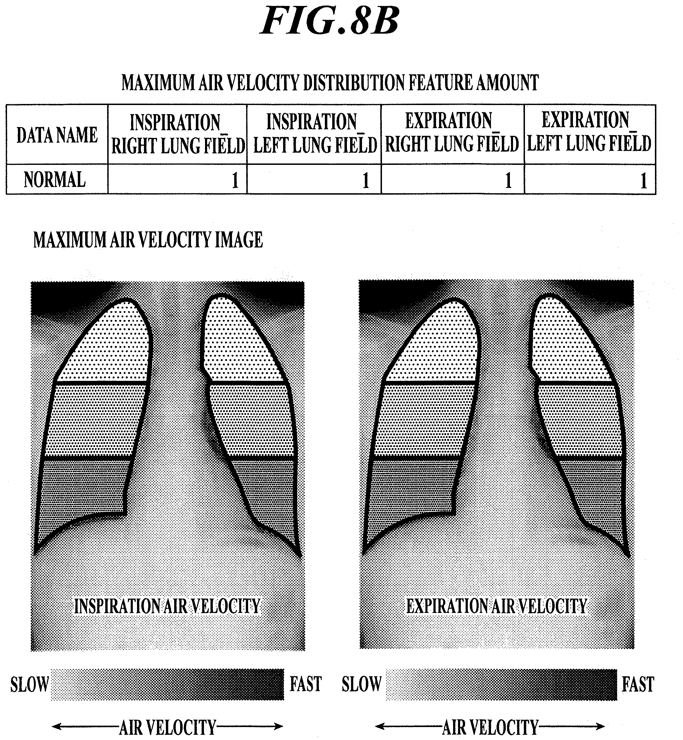

FIG. 8B is a diagram showing a display example of an analysis result displaying a maximum air velocity distribution feature amount and a maximum air velocity image for each block region;

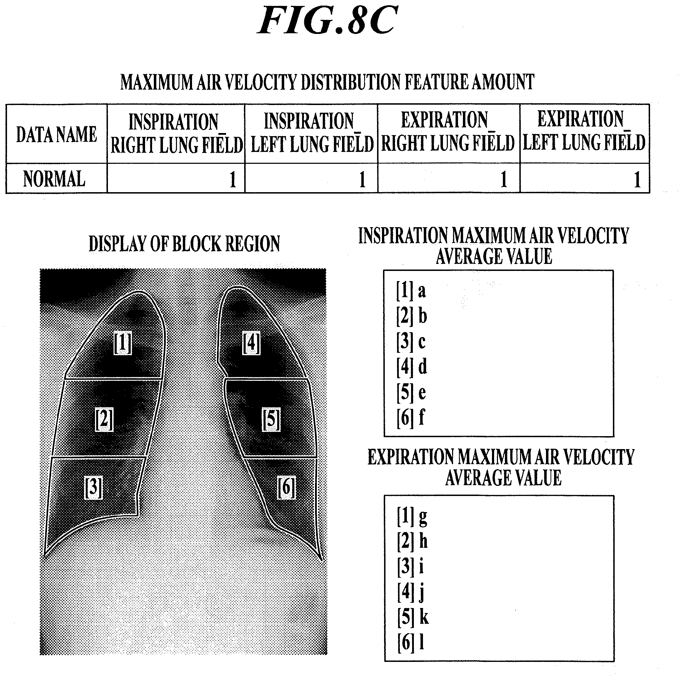

FIG. 8C is a diagram showing a display example of an analysis result displaying a maximum air velocity distribution feature amount and a numeric value of a maximum air velocity image for each block region;

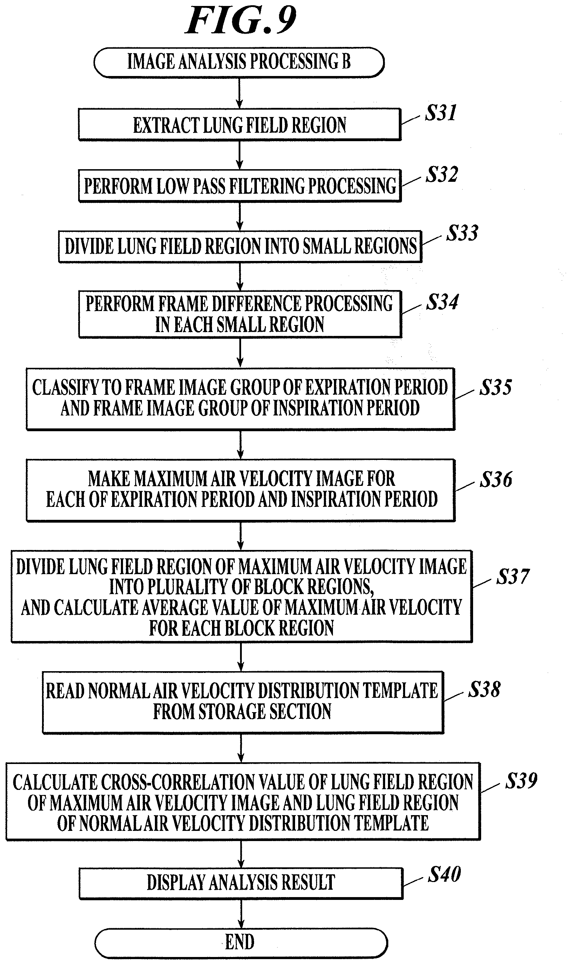

FIG. 9 is a flowchart showing image analysis processing B performed by a control section of a diagnosis console shown in FIG. 1;

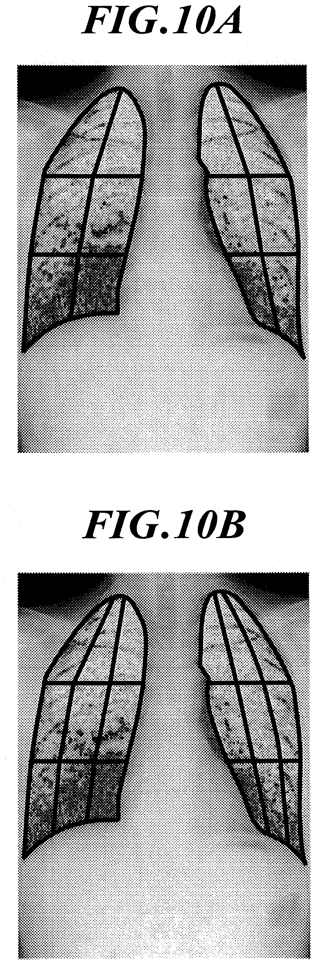

FIG. 10A is a diagram showing an example of dividing each of left and right lung field regions into a total of six block regions in a direction of a trunk axis direction and a direction substantially orthogonal to the trunk axis direction;

FIG. 10B is a diagram showing an example of dividing each of left and right lung field regions into a total of nine block regions in a direction of a trunk axis direction and a direction substantially orthogonal to the trunk axis direction;

FIG. 11 is a diagram showing an example of an expiration maximum air velocity image, an inspiration maximum air velocity image, an air velocity ratio image, and an air velocity ratio histogram of a normal lung field;

FIG. 12 is a diagram showing an example of an expiration maximum air velocity image, an inspiration maximum air velocity image, an air velocity ratio image, and an air velocity ratio histogram of a lung field of GOLD 3; and

FIG. 13 is a diagram showing an example of an expiration maximum air velocity image, an inspiration maximum air velocity image, an air velocity ratio image, and an air velocity ratio histogram of a lung field of GOLD 4.

DETAILED DESCRIPTION OF PREFERRED EMBODIMENTS

An embodiment of the present invention is described in detail with reference to the drawings. However, the scope of the claims is not limited to the illustrated examples.

First Embodiment

[Configuration of Thoracic Diagnosis Assistance System 100]

First, the configuration is described.

FIG. 1 shows an entire configuration of a thoracic diagnosis assistance system 100 of the present embodiment.

As shown in FIG. 1, the thoracic diagnosis assistance system 100 is configured by connecting a capturing apparatus 1 with a capturing console 2 through a communication cable, etc. and connecting a capturing console 2 with a diagnosis console 3 through a communication network NT such as a LAN (Local Area Network), etc. Each apparatus composing the thoracic diagnosis assistance system 100 complies to a DICOM (Digital Image and Communications in Medicine) standard, and communication between the apparatuses are performed according to DICOM.

[Configuration of Capturing Apparatus 1]

The capturing apparatus 1 is an apparatus to capture a state of the chest portion moving in cycles, such as change in shape from expansion and contraction of the lungs according to breathing movement, heartbeat, and the like. The dynamic state capturing is performed by successively emitting radiation such as X-rays on a chest portion of a human body to obtain a plurality of images (in other words, successive capturing). A string of images obtained by successive capturing is called a dynamic state image. Each of the plurality of images which compose the dynamic state image is called a frame image.

As shown in FIG. 1, the capturing apparatus 1 includes a radiation source 11, a radiation emitting control apparatus 12, a radiation detecting section 13, a reading control apparatus 14, and the like.

The radiation source 11 is provided in a position facing the radiation detecting section 13 with the subject M in between. According to control of the radiation emitting control apparatus 12, the radiation source 11 emits radiation (X-ray) on the subject M.

The radiation emitting control apparatus 12 is connected to the capturing console 2 and the radiation emitting control apparatus 12 controls the radiation source 11 to perform radiation capturing based on radiation emitting conditions input from the capturing console 2. The radiation emitting conditions input from the capturing console 2 are, for example, pulse rate, pulse width, and pulse interval of successive emitting, number of captured frames for each capturing, X-ray tube current value, X-ray tube voltage value, filter type, etc. The pulse rate is the number of times the radiation is emitted in one second and matches with a later described frame rate. The pulse width is the amount of time the radiation is emitted each time the radiation is emitted. The pulse interval is the amount of time from when emission of the radiation starts to when the next emission of the radiation starts in successive capturing. The pulse interval matches with a later described frame interval.

The radiation detecting section 13 is composed of a semiconductor image sensor such as a FPD, etc. The FPD includes, for example a glass substrate, etc. In the FPD, a plurality of pixels (detecting elements) are arranged in a matrix shape (two dimensionally) in a predetermined position on the substrate. The pixels detect radiation which is emitted from the radiation source 11 and which passes through at least the subject M according to the intensity, and converts the detected radiation into electric signals to be accumulated in the pixels. Each pixel is composed of a switching section such as a TFT (Thin Film Transistor), etc.

The reading control apparatus 14 is connected to the capturing console 2. The reading control apparatus 14 controls the switching section of each pixel of the radiation detecting section 13 based on image reading conditions input from the capturing console 2, switches reading of electric signals accumulated in each pixel, and reads the electric signals accumulated in the radiation detecting section 13 to obtain image data. The image data is the frame image. Then, the reading control apparatus 14 outputs the obtained frame image to the capturing console 2. The image reading conditions are, for example, frame rate, frame interval, pixel size, image size (matrix size), and the like. The frame rate is the number of frame images obtained in one second and matches with the pulse rate. The frame interval is the amount of time from when the operation of obtaining a frame image starts to when the operation of obtaining the next frame image starts in successive capturing. The frame interval matches with the pulse interval.

Here, the radiation emitting control apparatus 12 and the reading control apparatus 14 are connected to each other and the apparatuses transmit synchronizing signals between each other to synchronize the radiation emitting operation with the image reading operation. Moreover, in later described calibration where a plurality of dark images are obtained to calculate an offset correction coefficient used in offset correction, the image reading operation is not synchronized with the radiation emitting operation and a string of image reading operation which is reset - accumulate - read data - reset is performed in a state where radiation is not emitted. Here, the radiation can be emitted either before the string of dynamic state capturing or after the string of dynamic state capturing.

[Configuration of Capturing Console 2]

The capturing console 2 outputs the radiation emitting condition and the image reading condition to the capturing apparatus 1 and controls the radiation capturing and the reading operation of the radiation image by the capturing apparatus 1. The capturing console 2 also displays the dynamic image obtained by the capturing apparatus 1 so that the operator can confirm positioning or confirm whether or not the image is suitable for diagnosis.

As shown in FIG. 1, the capturing console 2 includes a control section 21, a storage section 22, an operation section 23, a display section 24, and a communication section 25 and each section is connected to each other through a bus 26.

The control section 21 includes a CPU (Central Processing Unit), a RAM (Random Access Memory), and the like. According to operation on the operation section 23, the CPU of the control section 21 reads a system program and various processing programs stored in the storage section 22 to be expanded in the RAM, and performs various processing such as later described capturing control processing according to the expanded program to centrally control operation of each section of the capturing console 2 and radiation emitting operation and reading operation of the capturing apparatus 1.

The storage section 22 includes a nonvolatile semiconductor memory, a hard disk and the like. The storage section 22 stores various programs performed by the control section 21, parameters necessary to perform processing by the program, or data of the processing result, etc. For example, the storage section 22 stores a capturing control processing program to perform the capturing control processing shown in FIG. 2. The storage section 22 stores the radiation emitting conditions and the image reading conditions to capture the dynamic state image of the chest portion. Various programs are stored in a state of a readable program code, and the control section 21 sequentially performs the operation according to the program code.

The operation section 23 is composed of a keyboard including a cursor key, a numeral input key, various function keys, etc., and a pointing device such as a mouse, etc. The operation section 23 outputs to the control section 21 instruction signals input by key operation on the keyboard or mouse operation. The operation section 23 can include a touch panel on the display screen of the display section 24, and in this case, the operation section 23 outputs to the control section 21 instruction signals input through the touch panel.

The display section 24 is configured with a monitor such as an LCD (Liquid Crystal Display), a CRT (Cathode Ray Tube) and the like. According to an instruction of display signals input from the control section 21, the display section 24 displays input instructions from the operation section 23, data and the like.

The communication section 25 includes a LAN adaptor, modem, a TA (Terminal Adapter), etc., and controls transmitting and receiving of data between the apparatuses connected to the communication network NT.

[Configuration of Diagnosis Console 3]

The diagnosis console 3 is a dynamic state image processing apparatus which obtains a dynamic state image from the capturing console 2, performs image analysis based on the obtained dynamic state image and displays the analysis result.

As shown in FIG. 1, the diagnosis console 3 includes a control section 31, a storage section 32, an operation section 33, a display section 34, and a communication section 35, and each section is connected to each other through a bus 36.

The control section 31 includes a CPU (Central Processing Unit), a RAM (Random Access Memory), and the like. According to operation on the operation section 33, the CPU of the control section 31 reads a system program and various processing programs stored in the storage section 32 to be expanded in the RAM, and performs various processing such as later described image analysis processing according to the expanded program to centrally control operation of each section of the diagnosis console 3. The control section 31 performs the later described image analysis processing to realize functions as an extracting section, an air velocity calculating section, and an air velocity distribution calculating section.

The storage section 32 includes a nonvolatile semiconductor memory, a hard disk and the like. The storage section 32 stores various programs performed by the control section 31 such as an image analysis processing program to perform image analysis processing, parameters necessary to perform processing by the program, or data of the processing result, etc. Various programs are stored in a state of a readable program code, and the control section 31 sequentially performs the operation according to the program code.

The operation section 33 is composed of a keyboard including a cursor key, a numeral input key, various function keys, etc., and a pointing device such as a mouse, etc. The operation section 33 outputs to the control section 31 instruction signals input by key operation on the keyboard or mouse operation. The operation section 33 can include a touch panel on the display screen of the display section 34, and in this case, the operation section 33 outputs to the control section 31 instruction signals input through the touch panel.

The display section 34 is configured with a monitor such as an LCD, a CRT and the like. According to an instruction of display signals input from the control section 31, the display section 34 displays input instructions from the operation section 33, data and the like.

The communication section 35 includes a LAN adaptor, modem, TA (Terminal Adapter), etc., and controls transmitting and receiving of data between the apparatuses connected to the communication network NT.

[Operation of Thoracic Diagnosis Assistance System 100]

Next, the operation of the thoracic diagnosis assistance system 100 is described.

(Operation of Capturing Apparatus 1, Capturing Console 2)

First, capturing operation by the capturing apparatus 1 and the capturing console 2 is described.

FIG. 2 shows capturing control processing performed by the control section 21 of the capturing console 2. The capturing control processing is performed by the control section 21 in coordination with the capturing control processing program stored in the storage section 22.

First, the operator operates the operation section 23 of the capturing console 2 and inputs patient information (patient name, height, weight, age, sex, etc.) of a capturing target (subject M) (step S1).

Next, the radiation emitting condition is read from the storage section 22 to be set in the radiation emitting control apparatus 12 and the image reading condition is read from the storage section 22 to be set in the reading control apparatus 14 (step S2).

Next, the process is put on standby for an instruction of emitting radiation with the operation of the operation section 23 (step S23). Here, the operator prepares for capturing with the capturing apparatus 1 such as positioning of the subject M, etc., and instructs the examinee (subject M) to relax to promote resting breathing in order to capture the dynamic state in resting breathing. When the preparation for capturing is complete, the operator operates the operation section 23 to input the radiation emitting instruction.

When the radiation emitting instruction is input on the operation section 23 (step S3; YES), the capturing start instruction is output to the radiation emitting control apparatus 12 and the reading control apparatus 14 to start the dynamic state capturing (step S4). In other words, the radiation is emitted from the radiation source 11 at the pulse interval set in the radiation emitting control apparatus 12, and the radiation detecting section 13 obtains the frame image. After capturing of a predetermined number of frames is finished, the control section 21 outputs an instruction to finish capturing to the radiation emitting control apparatus 12 and the reading control apparatus 14 and the capturing operation is terminated. The number of frames captured is at least a number of frames to be able to capture one breathing cycle.

The frame image obtained by capturing is sequentially input to the capturing console 2 and correction processing is performed on each frame image (step S5). In the correction processing of step S5, three correction processing are performed, which are offset correction processing, gain correction processing, and defective pixel correction processing. First, offset correction processing is performed on each obtained frame image and the offset value due to dark current overlapped on each obtained frame image is removed. For example, in the offset correction processing, the offset correction coefficient stored in advance is subtracted from each pixel value (density value, hereinafter referred to as signal value) of each obtained frame image. Here, the offset correction coefficient is the image averaging a plurality of frame images obtained in advance when radiation is not emitted. Next, the gain correction processing is performed to remove the variation of each pixel due to individual difference in each detecting element corresponding to each pixel of each frame image and gain unevenness of the reading amplifier. For example, in the gain correction processing, the gain correction coefficient stored in advance is multiplied to each frame image after offset correction. Here, the gain correction coefficient is a coefficient calculated and stored in advance so that the signal value of each pixel after correction becomes even, the coefficient calculated from the relation between the image averaging the plurality of offset corrected frame images obtained when the radiation is evenly emitted to the radiation detecting section 13 and the output signal value expected from the radiation emitting condition at this time. Next, the defective pixel correction processing is performed to remove the pixel with a non-linear sensitivity compared to the surrounding pixels or the defective pixel with no sensitivity. For example, in the defective pixel correction processing, according to a defective pixel position information map stored in advance, for each defective pixel registered in the defective pixel position information map, the signal value of each defective pixel is replaced with the average value of the signal values of the nearby pixels which are not defective. Here, the defective pixel position information map is a map registering in advance the position of a plurality of defective pixels acknowledged from the frame image after offset correction and gain correction obtained when radiation is emitted evenly on the radiation detecting section 13. For each of the above offset correction coefficient, gain correction coefficient, and defective pixel position information map, an optimum value is stored in advance according to acquisition mode such as binning, dynamic range, etc. and the corresponding optimum value is read for each acquisition mode.

Next, each frame image after correction processing is corresponded to a number showing an order of capturing to be stored in the storage section 22 (step S6) and displayed on the display section 24 (step S7). Here, logarithmic conversion processing to convert the signal value of each pixel of each frame image from an antilogarithm to a logarithm can be performed before storing each frame image. The operator confirms the positioning, etc. with the displayed dynamic state image, and determines whether an image suitable for diagnosis is obtained by capturing (capturing OK), or capturing needs to be performed again (capturing NG). Then, the operator operates the operation section 23 and inputs the judging result. Each frame image obtained by capturing can be collectively input after all capturing is finished.

It is also possible to successively display the frame images without performing the above correction processing when each frame image is successively transmitted to the console. With this, it is possible to promptly confirm the outline such as whether the positioning is suitable, whether at least one cycle of the dynamic state cycle of the subject is included, etc.

When the judging result showing that capturing is OK is input by the predetermined operation on the operation section 23 (step S8; YES), an identification ID to identify the dynamic state image, and information such as patient information, radiation emitting conditions, image reading conditions, a number showing capturing order, date and time of capturing, and the like are added to each of the string of frame images obtained by the dynamic state capturing (for example, writing in the header region of the image data in a DICOM format) and the frame image is transmitted to the diagnosis console 3 through the communication section 25 (step S9). Then, the processing ends. When the judging result showing capturing is NG is input by the predetermined operation on the operation section 23 (step S8; NO), the string of frame images stored in the storage section 22 is deleted (step S10), and the processing ends. In this case, capturing is performed again.

(Operation of Diagnosis Console 3)

Next, the operation of the diagnosis console 3 is described.

In the diagnosis console 3, when the string of frame images of the dynamic state image is received from the capturing console 2 through the communication section 35, the image analysis processing shown in FIG. 3 is performed by the control section 31 in coordination with the image analysis processing program stored in the storage section 32.

The flow of the image analysis processing is described with reference to FIG. 3.

First, a lung field region is extracted from a frame image which is to be a reference (reference image) (step S21).

It is preferable to use the frame image of the resting expiration level as the reference image. In the resting expiration level, the dimension of the lung field region becomes smallest in resting breathing. Therefore, if the frame image of the resting expiration level is used as the reference image, when each small region of the reference image is corresponded to another frame image, the small region is not corresponded to a region outside the lung field of the frame image.

The frame image of the resting expiration level can be obtained by extracting the image in which the position of the diaphragm is highest among the string of frame images. Alternatively, first the lung field region can be extracted from each frame image and then the frame image in which the dimension of the lung field region is smallest (the image in which the number of pixels in the lung field region is smallest) can be used as the reference image.

The method to extract the lung field region can be any method. For example, the threshold value can be obtained by discrimination analysis on the histogram of the signal value (density value) of each pixel of the frame image (reference image), and the region with the signal higher than the threshold value is first extracted as the lung field region candidate. Then, edge detecting is performed near a boundary of the lung field region candidate first extracted. If the point where the edge is largest in the small region near the boundary is extracted along the boundary, the boundary of the lung field region can be extracted.

Next, the low pass filtering processing in the time axis direction is performed (step S22). The lung field region appears as a change of the signal value by the movement of both ventilation and blood flow. The variation width of the signal value over time due to breathing ventilation is about ten times the variation width of the signal value due to lung blood flow. Therefore, when analyzing ventilation, the change of signal with high frequencies due to blood flow, etc. becomes noise. In order to remove the change of signals due to blood flow, etc., low pass filtering processing is performed at a cut off frequency of 1.0 Hz on the signal value which changes over time.

Next, the lung field region of each frame image is divided into a plurality of small regions and the small regions of each frame image are corresponded to each other (step S23). The position of the pixel of each small region is stored in the RAM of the control section 31.

Here, the breathing cycle is configured from an expiration period and an inspiration period. FIG. 4 is a diagram showing a frame image of a plurality of time phases (T=t0 to t6) captured in one breathing cycle (deep breathing). As shown in FIG. 4, in the expiration period, the diaphragm rises to discharge air from the lungs and the region of the lung field becomes small. In the maximum expiration level, the position of the diaphragm is in the highest state. In the inspiration period, the diaphragm lowers to take in air into the lungs, and as shown in FIG. 4, the region of the lung field in the thorax becomes large. In the maximum inspiration level, the position of the diaphragm is in the lowest state. In other words, the position of the same portion of the lung field region changes over time according to the breathing movement, and the pixel position showing the same portion of the lung field among frame images (specifically, lower region (near the diaphragm)) is displaced.

However, in the image captured in resting breathing, the displacement of the above position is small and displacement of position which causes the later described analysis result to become abnormal does not occur. Image D1 of FIG. 5 is a frame image of resting expiration level (timing when the position of the diaphragm is highest in resting breathing). Image D2 of FIG. 5 is a frame image of resting inspiration level (timing when the position of the diaphragm is lowest in resting breathing). In other words, the images D1 and D2 shown in FIG. 5 are images captured at the timing when the difference of shape is largest in one breathing cycle. However, it is possible to understand that the displacement of position is small between the images D1 and D2 of FIG. 5 in the lower region of the lung field region where displacement of position is largest (All of image D2 and A1 of image D1 show the same pixel position and A2 of image D2 and A1 of image D1 show the region drawing the same portion in the lung field).

In the specific processing of step S23, first, the lung field region extracted from the reference image is divided into a plurality of small regions (for example, rectangular regions of 2 mm × 2 mm) (see FIG. 5). Next, the lung field region of another frame image is divided into small regions with the same pixel position as the small regions of the reference image (region with the signal value output from the same detecting element of the radiation detecting section 13). Next, the small regions with the same pixel position are corresponded among the frame images. The lung field region of each frame image is to be the region with the same pixel position as the reference image (region including the plurality of small regions). With this processing, it is possible to perform dividing and corresponding of the small regions of the frame image with high speed.

Here, the dynamic state image of resting breathing is used, however, when the captured dynamic state image is an image of deep breathing, as shown in FIG. 6, the pixel position showing the same portion of the lung field is drastically displaced (A31 of image D4 and A3 of image D3 show the same pixel position and A4 of image D4 and A3 of image D3 show the region drawing the same position in the lung field). Therefore, if the region in each frame image with the same pixel position as each small region in the reference image is set as the region corresponding to the small region similar to resting breathing, the later described analysis result cannot be used in diagnosis. Therefore, in such case, corresponding point extracting processing (local matching processing) which extracts the corresponding points among the frame images and nonlinear distortion conversion processing (warping processing) are performed, and the regions drawing the same portion of the lung field region are corresponded to each other among the frame images. Moreover, in resting breathing also, if the processing speed is not emphasized and it is desired to enhance analysis accuracy, the above processing can be performed.

In the local matching processing, first the lung field region is extracted from the frame image with number 1 (first) in the capturing order, and the extracted lung field region is divided into small regions such as 2 mm rectangles.

Next, the frame image with number 1 in the capturing order is set to PI, the adjacent frame image (the frame image with the adjacent capturing order (in other words, adjacent frame image in terms of time, similar hereinafter)) is set to P2, and the search region for each small region of P1 is set in P2. Here, the search region is the region which has the same center point $(x, y)$ when the coordinates of the center point in each small region of P1 is $(x, y)$, and the dimension is set larger than the small region P1 vertically and horizontally (for example, 1.5 times). Then, for each region of P1, the region with the highest matching degree among the search range of the P2 is obtained, and the position in P2 corresponding to each small region of P1 is calculated. In order to find the matching degree, a least squares method or a cross-correlation coefficient is used as an index. Then, the lung field region of P2 is divided at the position corresponding to each small region of P1.

Then, P2 is newly considered to be PI, the frame image with the next capturing order after P2 is newly considered to be P2, and the position of P2 corresponding to each small region of P1 is calculated. The above processing is repeated to obtain the position of the adjacent frame image which corresponds to each small region of each frame image. The obtained processing result is stored in the RAM of the control section 31.

Next, the warping processing is performed. Specifically, the frame image with number 1 in the capturing order is set to PI, the frame image with the adjacent capturing order (adjacent in terms of time) is set to P2, and a shift vector from P1 to P2 is calculated for each small region based on the corresponding position of each small region between the adjacent frame images calculated in the local matching processing. Next, the calculated shift vector is fit with a polynomial expression, and the polynomial expression is used to calculate the shift vector of each pixel in each small region. Then, the warping processing is performed based on the calculated shift vector of each pixel, and the position of each pixel in each small region of P2 is shifted to the position of the pixel corresponding to the frame image of P1. Next, P2 on which warping processing is performed is newly considered to be PI, the frame image with the next capturing order after P2 is newly considered to be P2, and the above processing is performed. The above processing is repeated sequentially from the adjacent frame images with the early capturing order, so that it is possible to substantially match the position of each small region of all frame images to the frame image with the capturing order 1 (the reference image of this processing). The corresponding relation of the position of the small region among the frame images is stored in the RAM of the control section 31.

Next, frame difference processing is performed on each small region of the string of frame images corresponded in step S23 and the frame difference value is calculated (step S24).

The frame difference value is a value showing a signal variation amount in the timing that the frame image is captured. When breath is inhaled or exhaled by breathing, the density of the lungs changes according to the flow of breathing, and with this, the X-ray transmission amount (in other words, the pixel output signal value) changes. Therefore, it can be said that the signal variation amount is a value showing the air velocity at the certain timing (feature amount showing the air velocity).

Specifically, the signal value (average signal value) of the pixel in each small region in each frame image is calculated. Next, the frame difference processing is performed to calculate the difference of the signal value of each small region between the frame images with adjacent capturing order. Here, the difference value of N+1-N is calculated for each small region of frame images with frame numbers of N and N+1 (N is 1, 2, 3 and so on).

Next, classifying between the frame image group of the inspiration period and the frame image group of the expiration period is performed (step S25). The period in which the sign of the frame difference value in each small region is positive is to be the inspiration period and the period in which the sign of the frame difference value in each small region is negative is to be the expiration period.

Next, the maximum value (maximum value of the absolute value) of the frame difference value in the expiration period in each small region is obtained as the expiration maximum air velocity, and the expiration maximum air velocity image is made with the value of each small region as the expiration maximum air velocity. Moreover, the maximum value (maximum value of the absolute value) of the frame difference value in the inspiration period in each small region is obtained as the inspiration maximum air velocity, and the inspiration maximum air velocity image is made with the value of each small region as the inspiration maximum air velocity (step S26).

Next, in each expiration maximum air velocity image and inspiration maximum air velocity image, the lung field region is divided in a plurality of block regions in a trunk axis direction (head-foot direction, upper and lower direction) and a representative value (here, average value) of the maximum air velocity in each block region is calculated (step S27). The representative value of the maximum air velocity in each block region is the feature amount showing the air velocity of each block region.

For example, the lung field region can be divided by dividing each of the left lung region and right lung region into three block regions of upper block region, middle block region, and lower block regions, which are to be block regions L1 to L3, R1 to R3, as shown in FIG. 7.

Here, the average value is used as the representative value of the maximum air velocity in each block region. However, the representative value is not limited to the average value, and a minimum value, a maximum value, a medium value, or an integrated value may be used.

Then, the difference value of the maximum air velocity average value is calculated between the adjacent block regions in the trunk axis direction in each of the expiration maximum air velocity image and the inspiration maximum air velocity image (maximum air velocity average value of the lower block region - maximum air velocity average value of the upper block region). Based on the calculated difference value, the feature amount representing the maximum air velocity distribution of the lung field in the trunk axis direction (called the maximum air velocity distribution feature amount) is calculated (step S28).

The maximum air velocity distribution feature amount is calculated in expiration and inspiration for each of the left and right lung fields according to formula 1 described below. It is also possible to use the formula 1 to calculate the maximum air velocity distribution feature amount for the entire lung field in addition to each of the left and right lung fields.

FORMULA 1

$$\text{FEATURE AMOUNT} = \frac{\text{DIFFERENCE VALUE AMONG ADJACENT BLOCK REGIONS} > \text{NUMBER OF PIECES OF DATA WITH } 0}{\text{NUMBER OF DIVISION OF ADJACENT BLOCK REGIONS}}$$

After calculating the feature amount, the analysis result (including maximum air velocity distribution feature amount) is displayed on the display section 34 (step S29).

In a normal lung field, in both expiration and inspiration, the air velocity gradually decreases towards the upper lung field. In a lung field with a ventilation disease such as emphysema, such tendency is lost as the degree of severity of the disease becomes heavier. In other words, in a normal lung field, substantially all of the values of the difference value of the air velocity between adjacent block regions in the trunk axis direction (maximum air velocity average value of the lower block region - maximum air velocity average value of the upper block region) are larger than 0, and the calculated value of the maximum air velocity distribution feature amount becomes about 0.9 or more. When the lung field is abnormal, a negative value is mixed in the value of the difference value of the air velocity between the adjacent block regions and the calculated value of the maximum air velocity distribution feature value becomes smaller than 0.9. The severer the abnormality is, the smaller the calculated value of the feature amount becomes.

As described above, a doctor is able to easily acknowledge a ventilation disease even if there is no difference between the inspiration air velocity and the expiration air velocity by using the maximum air velocity distribution feature amount showing the maximum air velocity distribution in the trunk axis direction of the lung field and this can be used as material in determining a disease for diagnosis.

FIG. 8A to FIG. 8C show a display example of an analysis result in step S29. As the analysis result, other than the maximum air velocity distribution feature amount for each of expiration and inspiration, it is preferable to display the maximum air velocity image. By displaying the maximum air velocity image, it is possible for the physician to easily (intuitively) acknowledge the actual distribution of the maximum air velocity of each lung field.

As shown in FIG. 8A, the maximum air velocity image can be an image showing each small region on the frame image (reference image) divided by chroma (or luminance) according to the maximum air velocity. Alternatively, as shown in FIG. 8B, the maximum air velocity image can be an image showing each block region on the frame image (reference image) divided by chroma (or luminance) according to the average value of the maximum air velocity. It is preferable to display the corresponding relation between the maximum air velocity and the chroma (or luminance) near the maximum air velocity image. Moreover, as shown in FIG. 8C, in the maximum air velocity image, the average value of the maximum air velocity of each block region is displayed with a numeric value. In FIG. 8C, a to f each show a numeric value representing the average value of the maximum air velocity of the block regions [1] to [6].

It is preferable to display both the expiration maximum air velocity image and the inspiration air velocity image. However, it is possible to display either one with the worse result.

In step S29, it is preferable to calculate (make) and display a histogram of the air velocity ratio (inspiration maximum air velocity/ expiration maximum air velocity) for each small region and an index value (average value, standard deviation, half value width, etc.) showing a tendency of air velocity ratio in the entire lung field (see 34d of FIG. 11 to FIG. 13). By using the maximum air velocity distribution feature amount, it is possible to discriminate a ventilation disease where a difference cannot be seen between the inspiration air velocity and the expiration air velocity and by displaying the histogram and the index value of the air velocity ratio, it is possible to provide diagnosis assistance information for a ventilation disease where a difference between the inspiration air velocity and the expiration air velocity is large. An image showing each small region on the frame image (reference image) divided by chroma (or luminance) according to the air velocity ratio can be displayed together with the histogram, etc. (see 34a, 34b of FIG. 11 to FIG. 13). With this, it is possible for the physician to easily acknowledge regional abnormal portions in the lung field with a large air velocity ratio.

In step S29, either one or both of the original moving image and ventilation moving image can be displayed. By displaying the above, it is possible for the physician to observe the actual movement of the lungs together with the analysis result. The original moving image sequentially switches display of the frame image of the dynamic state image captured by the capturing apparatus 1 (flipping display). The ventilation moving image sequentially switches display of the frame image on which the low pass filtering is performed in step S22.

According to the first embodiment, each of the right lung field and the left lung field are divided into three block regions in the trunk axis direction. However, as shown in FIG. 7B, the lung fields can be divided in three block regions or more. By dividing the block regions into smaller regions, it is possible to enhance accuracy of the maximum air velocity feature amount. As a tendency of the normal air velocity, there is a difference in tendency of the air velocity between the inside and the outside. Therefore, as shown in FIG. 7C, it is possible to consider the change of the air velocity from the inside to the outside and to divide the lung field regions into block regions according to clinical practice. With this, it is possible to enhance accuracy of calculation of the maximum air velocity feature amount.

According to the first embodiment, the air velocity ratio (inspiration maximum air velocity/ expiration maximum air velocity) in each small region is used as the feature amount to make the histogram and to calculate the index value (average value, standard deviation, half value width, etc.) showing the tendency of the air velocity ratio in the entire lung field. Alternatively, as the feature amount, it is possible to use the result of adding, subtracting and multiplying the inspiration maximum air velocity and the expiration maximum air velocity. Moreover, it is also possible to use only either one of the inspiration maximum air velocity or the expiration maximum air velocity to make the histogram and to calculate the index value (average value, standard deviation, half value width, etc.) showing the tendency of the air velocity ratio in the entire lung field. With this, although the accuracy is slightly less than the above embodiment, it is possible to see an abnormal function of the dynamic state (ventilation function) which is the capturing target.

Second Embodiment

Next, the second embodiment is described.

The second embodiment is different from the first embodiment in that the template of the maximum air velocity distribution for each of expiration and inspiration of the normal lungs (normal air velocity distribution template) is stored in the storage section 32 of the diagnosis console 3. The content of the image analysis processing performed by the control section 31 of the diagnosis console 3 is also different. Other than the above, the entire configuration of the thoracic diagnosis assistance system 100, the configuration of each apparatus and the operation of the capturing apparatus 1 and the capturing console 2 are similar to those as described in the first embodiment and the description is to be referred.

The image analysis processing (image analysis processing B) of the second embodiment is described.

FIG. 9 shows a flowchart of image analysis processing B of the second embodiment. The image analysis processing B is performed by the control section 31 in coordination with the image analysis processing B program stored in the storage section 32.

First, the processing of steps S31 to S37 are performed to make the maximum air velocity image for each of the expiration period and the inspiration period from the string of frame images, the lung field regions are divided into a plurality of block regions in the trunk axis direction and the representative value (here, average value) of the maximum air velocity of each block region is calculated. The processing of step S31 to step S37 are similar to the processing of step S21 to step S27, and therefore the description is referred.

Next, the normal air velocity distribution template is read for each of the expiration and the inspiration from the storage section 32 (step S38).

Here, there is a tendency in the air velocity of the lung field with a normal ventilation function that the air velocity gradually decreases from the lower lung field to the upper lung field. The image assigned with the various values of the maximum air velocity in each block region can be used in the normal air velocity distribution template so as to meet this tendency. For example, the following values can be assigned to the region L1 (R1), L2 (R2), and L3 (R3) shown in FIG. 7A, L1 (R1)=3, L2 (R2)=2, L3 (R3)=1.

Alternatively, the processing similar to the above steps S31 to S37 can be performed in advance on a plurality of dynamic state images of the lung fields of those of a plurality of people with normal lungs, the average value of the maximum air velocity of each block region can be calculated, and the average of the above can be used as the normal air velocity distribution template.

The maximum air velocity image and the block region of the normal air velocity distribution template are corresponded to each other. A plurality of the normal air velocity distribution templates can be stored in the storage section 32 according to the following conditions, and the normal air velocity distribution template can be read and used according to the input from the operation section 33 or the patient information.

Conditions: sex, age, waist circumference, chest circumference, height, weight, lung capacity, surgical history (use of pacemaker, history of excision of lungs, etc.), breathing when capturing (resting breathing, deep breathing (forced breathing)), history of past disease, etc.

Next, the cross-correlation value (cross-correlation coefficient) between the maximum air velocity image and the normal air velocity distribution template is calculated for each of the right lung field of the expiration, the left lung field of the expiration, the right lung field of the inspiration and the left lung field of the inspiration using the maximum air velocity image of each lung field and the value of the normal air velocity distribution template (step S39). The cross-correlation value of each lung field shows the correlation between the maximum air velocity distribution of each lung field and the normal air velocity distribution and this is to be the feature amount showing the distribution of the maximum air velocity in each lung field (maximum air velocity distribution feature amount).

In step S39, the processing calculates four cross-correlation values composed of the cross-correlation value of the expiration right lung field, the cross-correlation value of the expiration left lung field, the cross-correlation value of the inspiration right lung field, and the cross-correlation value of the inspiration left lung field, and the product of the four cross-correlation values.

For example, the cross correlation value of the expiration right lung field can be obtained by the formula 2 using the average value of the maximum air velocity of each block region of the right lung field in the expiration maximum air velocity image and the average value of each block region of the right lung field in the expiration normal air velocity distribution template (for example, see publicly known document 1: Mikio TAKAGI, Haruhisa SHIMODA, "New Edition Image Analysis Handbook", University of Tokyo Press, 2004).

FORMULA 2

$$C = \frac{1}{J} \sum_{j=1}^{J} \frac{\{A(j) - m_A\}\{B(j) - m_B\}}{\sigma_A \; \sigma_B}$$

$$m_A = \frac{1}{J} \sum_{j=1}^{J} A(j), \quad m_B = \frac{1}{J} \sum_{j=1}^{J} B(j)$$

$$\sigma_A = \sqrt{\frac{1}{J} \sum_{j=1}^{J} \{A(j) - m_A\}^2}$$

$$\sigma_B = \sqrt{\frac{1}{J} \sum_{j=1}^{J} \{B(j) - m_B\}^2}$$

C : CROSS-CORRELATION VALUE
A(j): AVERAGE VALUE OF j-TH REGION AMONG TOTAL NUMBER OF J REGIONS INCLUDED IN MAXIMUM AIR VELOCITY IMAGE
$m_A$ : AVERAGE SIGNAL VALUE OF ALL REGIONS INCLUDED IN MAXIMUM AIR VELOCITY IMAGE
$\sigma_A$ : STANDARD DEVIATION OF ALL REGIONS INCLUDED IN MAXIMUM AIR VELOCITY IMAGE
B(j) : AVERAGE VALUE OF j-TH REGION AMONG TOTAL NUMBER OF J REGIONS INCLUDED IN TEMPLATE
$m_B$ : AVERAGE SIGNAL VALUE OF ALL REGIONS INCLUDED IN TEMPLATE
$\sigma_B$ : STANDARD DEVIATION OF ALL REGIONS INCLUDED IN TEMPLATE

Here, j=1, 2 and so on are applied in order from the lower lung region.

In other words, the cross correlation value of the maximum air velocity of the expiration right lung field can be obtained by, first calculating the covariance between the average value of the maximum air velocity of each block region of the right lung field in the expiration maximum air velocity image and the average value of each block region of the right lung field in the expiration normal air velocity distribution template, and then dividing the calculated covariance with the product of the standard deviation of the average value of the expiration maximum air velocity of each block region of the right lung field in the expiration maximum air velocity image and the standard deviation of the average value of each block region of the right lung field in the expiration normal air velocity distribution template.

The calculating method is the same for the cross-correlation value of the expiration left lung field, the cross-correlation value of the inspiration right lung field, and the cross-correlation value of the inspiration left lung field by replacing the portion of the expiration right lung field with that of the expiration left lung field, the inspiration right lung field and the inspiration left lung field, and changing the maximum air velocity image and the normal air velocity distribution template to be used to those which correspond.

As the feature amount, instead of using the cross-correlation value for each lung field, it is possible to obtain the degree of similarity by SSDA method described in the above publicly known document.

In a normal lung field, the air velocity gradually reduces toward the upper lung field in both expiration and inspiration. In a lung field with a ventilation disease such as emphysema, such tendency is lost as the degree of severity of the disease becomes heavier. Therefore, when the cross-correlation value between the maximum air velocity image and the normal air velocity distribution template is obtained for each lung field, the cross-correlation value of the normal lung field is close to 1, and the severer the abnormality is, the value becomes further from 1. The severer the abnormality of the lung field is, the product of each lung field becomes a value further from 1. As described above, by displaying the maximum air velocity distribution feature amount showing the maximum air velocity distribution in the trunk axis direction of the lung field, the physician is able to easily acknowledge a ventilation disease even if there is no difference between the inspiration air velocity and the expiration air velocity.

After calculating the feature amount, the analysis result (including maximum air velocity distribution feature amount) is displayed on the display section 34 (step S40).

Similar to the analysis result of the first embodiment, the analysis result in step S40 can also be displayed as shown in FIG. 8A to FIG. 8C. In other words, other than the maximum air velocity distribution feature amount, it is preferable to display the maximum air velocity image. Moreover, it is preferable to calculate (make) and display a histogram of the air velocity ratio (inspiration maximum air velocity/ expiration maximum air velocity) for each small region and an index value (average value, standard deviation, half value width, etc.) showing a tendency of air velocity ratio in the entire lung field (see 34d of FIG. 11 to FIG. 13). An image showing each small region on the frame image (reference image) divided by chroma (or luminance) according to the air velocity ratio can be displayed together with the histogram, etc. (see 34a, 34b of FIG. 11 to FIG. 13). Either one or both of the original moving image and ventilation moving image can be displayed.

In step S40, the analyzed normal air velocity distribution template can also be displayed. For example, the normal air velocity distribution template can be displayed as an image showing the lung fields divided by chroma (or luminance) according to the value of the normal air velocity distribution template or the value of each block region of the normal air velocity distribution template can be displayed with a numeric value.

It is preferable to display the normal air velocity distribution template of both the expiration and the inspiration, however, only either one with the worse result can be displayed.

In step S40, it is possible to calculate and display the "absolute value of difference" or "square value of difference" for each corresponding block region between the maximum air velocity image and the normal air velocity distribution template. With this, it is possible for the physician to acknowledge which block region is abnormal. It is preferable to display the "absolute value of difference" or "square value of difference" of both the expiration and the inspiration, however, only either one with the worse result can be displayed. Before obtaining the difference, the value can be normalized by dividing the value by an average value, a maximum value, a minimum value, or a medium value in order to match the level between the maximum air velocity image and the normal air velocity distribution template image.

When dividing the lung field regions into block regions, other than dividing each lung field into three block regions composed of upper block region, middle block region, and lower block region as described above, it is possible to divide the lung field into three block regions or more in the trunk axis direction as shown in FIG. 7B, similar to the first embodiment. As shown in FIG. 7C, the lung fields can be divided into block regions according to clinical practice. As shown in FIG. 10A and FIG. 10B, it is possible to divide the lung field region into a plurality of block regions in the trunk axis direction and a direction substantially orthogonal to this direction. With this, it is possible to calculate the feature amount considering the distribution of the air velocity from inside to outside the lung field in addition to the distribution of the air velocity of the lung field in the trunk axis direction.

Described below is the testing result of effectiveness of the maximum air velocity distribution feature amount (cross-correlation value) calculated by the image analysis processing B.

Chart 1 and chart 2 show the cross-correlation value of the normal lung field shown in FIG. 11 calculated in the image analysis processing B (upper line) and the cross-correlation value of the lung field with emphysema (GOLD 3) shown in FIG. 12 calculated in the image analysis processing B (lower line). As shown in FIG. 12, the lung field with emphysema shows a result which appears to be normal in analysis using the air velocity ratio.

Chart 1 shows a case where each lung field is divided into three block regions of upper block region, middle block region, and lower block region as shown in FIG. 7A. Chart 2 shows a case where each lung field is divided into twenty block regions as shown in FIG. 7B. In either case, the cross-correlation value is calculated using the template made based on the tendency of the air velocity of the lung field with a normal ventilation function. When the cross-correlation value is less than 0, all of the values are set to 0.

Table 1

| DATA NAME | CROSS-CORRELATION VALUE | | | | |
|---|---|---|---|---|---|
| | INSPIRATION_ RIGHT LUNG FIELD | INSPIRATION_ LEFT LUNG FIELD | EXPIRATION_ RIGHT LUNG FIELD | EXPIRATION_ LEFT LUNG FIELD | PRODUCT OF EACH CORRELATION RESULT |
| NORMAL | 0.99 | 0.98 | 0.99 | 0.99 | 0.96 |
| EMPHYSEMA | 0.92 | 0.98 | 0.98 | 0.87 | 0.78 |

Table 2

| | CROSS-CORRELATION VALUE | | | | |
|---|---|---|---|---|---|
| DATA NAME | INSPIRATION_ RIGHT LUNG FIELD | INSPIRATION_ LEFT LUNG FIELD | EXPIRATION_ RIGHT LUNG FIELD | EXPIRATION_ LEFTLUNG FIELD | PRODUCT OF EACH CORRELATION RESULT |
| NORMAL | 0.95 | 0.96 | 0.96 | 0.98 | 0.87 |
| EMPHYSEMA | 0.58 | 0.55 | 0.65 | 0.58 | 0.12 |

As shown in chart 1, when each of the left and right lung fields are divided into three block regions and the cross-correlation value with the normal air velocity distribution template is calculated, the values of the normal lung field as described in the upper line are, 0.99 for the inspiration right lung field, 0.98 for the inspiration left lung field, 0.99 for the expiration right lung field, and 0.99 for the expiration left lung field. All of the cross-correlation values are values close to 1. The values of the lung field with emphysema as described in the lower line are, 0.92 for the inspiration right lung field, 0.98 for the inspiration left lung field, 0.98 for the expiration right lung field, and 0.87 for the expiration left lung field. The product of the cross-correlation values for each of the normal lung field and the lung field with emphysema is, 0.96 for the normal lung field and 0.78 for the lung field with emphysema. The result shows an outstanding difference between the value of the normal lung field and the value of the lung field with emphysema.

As shown in chart 2, when each of the left and right lung fields are divided into twenty block regions and the cross-correlation value with the normal air velocity distribution template is calculated, the values of the normal lung field as described in the upper line are, 0.95 for the inspiration right lung field, 0.96 for the inspiration left lung field, 0.96 for the expiration right lung field, and 0.98 for the expiration left lung field. All of the cross-correlation values are values close to 1. The values of the lung field with emphysema as described in the lower line are, 0.58 for the inspiration right lung field, 0.55 for the inspiration left lung field, 0.65 for the expiration right lung field, and 0.58 for the expiration left lung field and all of the values are near 0.6. The product of the cross-correlation values for each of the normal lung field and the lung field with emphysema is, 0.87 for the normal lung field and 0.12 for the lung field with emphysema. The result shows an outstanding difference between the value of the normal lung field and the value of the lung field with emphysema.

According to consideration by the inventors of the present application, an indication of a normal lung field is the product of the cross-correlation being 0.9 or more when each of the left and right lung fields are divided into about three block regions and 0.8 or more when each of the left and right lung fields are divided into about twenty block regions. As the degree of severity of ventilation progresses, the product becomes close to 0. In both of the above chart 1 and chart 2, considering the product of each cross-correlation value, the value is the indication or more in the normal lung field and the value is less than the indication in the lung field with emphysema and it can be said that the cross-correlation value is effective for diagnosis. Therefore, by generating and outputting the cross-correlation value which is the maximum air velocity distribution feature amount, it is possible to provide material to judge whether the lung field of the analysis target is normal or not. Comparing the result of the chart 1 in which each of the left and right lung fields are divided into three block regions and the result of the chart 2 in which each of the left and right lung fields are divided into twenty block regions, the values of the cross-correlation values between the normal lung field and the lung field with emphysema are further apart when divided into twenty block regions, and the result clearly shows that there is an abnormality.

In order to evaluate the effectiveness of the maximum air velocity distribution feature amount, the maximum air velocity distribution feature amount (cross-correlation value) is calculated by the image analysis processing B for samples in which the degree of air flow limit of COPD is classified by the spirometeric examination result in advance (normal: 29 people, GOLD 1 or 2: 20 people, GOLD 3 or 4: 31 people (GOLD 3: 23 people, GOLD 4: 8 people)). Then, the value of the maximum air velocity distribution feature amount is judged whether there is a statistical significant difference among the groups by evaluation using the Wilcoxon test. The Wilcoxon test is a well known statistical method to evaluate effectiveness of data (well known document 2: Kiyoshi ICHIHARA, "Statistics in Bio-Science", NANKODO, 1990). In the Wilcoxon test, the values are considered to have a significant difference when the value is less than 0.05.

Chart 3 shows a result of the Wilcoxon test regarding cross-correlation values obtained by dividing each of the left and right lung fields to three block regions of upper block region, middle block region, and lower block region as shown in FIG. 7A, and calculating the values using a normal air velocity distribution template made based on the tendency of air velocity of a lung field with a normal ventilation function. In this case, it is possible to see a significant difference between the cross-correlation value of the normal lung field and the cross-correlation value of the lung field of GOLD 3 or GOLD 4.

Table 3

|  | NORMAL | GOLD1&2 |
|---|---|---|
| GOLD1&2 | 0.06187 | - |
| GOLD3&4 | 0.00051 | 0.08439 |

Chart 4 shows a result of the Wilcoxon test regarding cross-correlation values obtained by dividing each of the lung fields to twenty block regions as shown in FIG. 7B, and calculating the values using a normal air velocity distribution template made based on the tendency of the air velocity of the lung field with the normal ventilation function. In this case, it is possible to see a significant difference between normal and GOLD 3, normal and GOLD 4, GOLD 1 or 2 and GOLD 4, and GOLD 3 and GOLD 4.

Table 4

|  | NORMAL | GOLD1&2 | GOLD3 |
|---|---|---|---|
| GOLD1&2 | 0.08439 | - | - |
| GOLD3 | 0.00027 | 0.08439 | - |
| GOLD4 | 0.00006 | 0.00119 | 0.02595 |

As described above, according to the thoracic diagnosis assistance system 100, when a plurality of frame images generated by capturing the dynamic state of the chest portion is input from the capturing console 2 through the communication section 35, the control section 31 of the diagnosis console 3 extracts the lung field region from one of the frame images among the plurality of frame images, divides the extracted lung field region into a plurality of small regions, corresponds the small regions among the plurality of frame images, and calculates the feature amount showing the air velocity for each of the small regions. Then, the lung field regions are divided into a plurality of block regions in the trunk axis direction, the feature amount showing the air velocity of each block region is calculated based on the feature amount showing the air velocity of the plurality of small regions included in the divided block regions, and the feature amount showing the distribution of the air velocity of the lung field region in the trunk axis direction is calculated based on the calculated feature amount showing air velocity of each small region.

In a normal lung field, the air flow velocity reduces from the lower lung field toward the upper lung field. In a lung field with a ventilation disease such as emphysema, such tendency is lost as the degree of severity of the disease becomes heavier. Therefore, by calculating the feature amount showing the distribution of the air velocity of the lung field region in the trunk axis direction, it is possible to provide diagnosis assistance information so that it is possible for a physician to easily acknowledge a ventilation disease even if there is no difference between the inspiration air velocity and the expiration air velocity.

Each of the left and right lung field regions are divided into a plurality of block regions in the trunk axis direction, and the feature amount showing the distribution of the air velocity of the trunk axis direction is generated for each of the left and right lung field regions. Therefore, it is possible to provide diagnosis assistance information so that the physician can easily acknowledge the tendency of distribution of air velocity in each of the left and right lung field regions in the trunk axis direction.

By dividing the lung field region into three or more block regions in the trunk axis direction and calculating the feature amount showing the distribution of the air velocity of the lung field region in the trunk axis direction, it is possible to provide diagnosis assistance information which accurately shows whether or not the distribution of the air velocity in the trunk axis direction is monotonically decreasing toward the upper portion of the lung field.

By dividing the lung field region into a plurality of block regions in the trunk axis direction and the direction substantially orthogonal to such direction, it is possible to provide diagnosis assistance information considering the distribution of air velocity of the lung field from the inside to the outside in addition to the distribution of the air velocity of the lung field in the upper and lower direction.

For example, as the feature amount showing the distribution of the air velocity of the lung field region in the trunk axis direction, it is possible to calculate the cross-correlation value between the air velocity of the lung field region and the template showing the air velocity distribution of the normal lung field. With this, it is possible to provide information of whether the air velocity distribution of the lung field region has a strong correlation with a normal state by a numeric value.

The description of the above embodiment is one example of a suitable thoracic diagnosis assistance system of the present invention and the present invention is not limited to the above.

For example, the above described description describes an example using a hard disk, a nonvolatile memory, etc. as a computer readable medium of the program regarding the present invention, however, the present invention is not limited to the above example. As other computer readable mediums, it is possible to apply a portable storage medium such as a CD-ROM, etc. Moreover, as the medium providing data of the program regarding the present invention through the communication line, a carrier wave can be applied.

Other than the above, the detailed configuration and the detailed operation of each apparatus composing the thoracic diagnosis assistance system 100 can be suitably modified without leaving the scope of the present invention.

**Claims**

1. A thoracic diagnosis assistance information generation method comprising:

    capturing (S4) a dynamic state of a chest portion including at least one breathing cycle using a detector (13) with detecting elements aligned two dimensionally to generate a plurality of successive frame images;
    extracting (S21) a lung field region from one frame image among the plurality of frame images generated in the capturing;
    calculating (S28) air velocity by dividing the lung field region extracted in the extracting into a plurality of small regions, corresponding the small regions among the plurality of frame images, performing analysis for each corresponded small region, and calculating a feature amount showing air velocity for each small region; and calculating (S28) air velocity distribution by dividing (S23) the lung field region into a plurality of block regions in a trunk axis direction, calculating a feature amount showing air velocity of each block region based on a feature amount showing (S29) the air velocity of the plurality of small regions included in the divided block regions, and calculating a feature amount showing air velocity distribution of the lung field region in the trunk axis direction based on the calculated feature amount showing the air velocity of each block region, wherein calculating the air velocity distribution includes reading from a storage section a template showing air velocity distribution of a normal lung field and calculating as a feature amount showing air velocity distribution of the lung field region in the trunk axis direction, a degree of match of air velocity distribution between the lung field region and a lung field region in the template, based on a feature amount showing air velocity of the plurality of block regions and a feature amount showing air velocity in each corresponding region in the template, and wherein the calculating (S28) of the air velocity distribution includes calculating cross-correlation coefficient (S39) of the lung field region and the lung field region of the template as the degree of match of the air velocity distribution between the lung field region and the lung field region of the template.

2. The thoracic diagnosis assistance information generation method of claim 1, wherein calculating (S28) the air velocity distribution includes dividing (S27) each of a left lung field region and a right lung field region into a plurality of block regions (L1, L2, L3, R1, R2, R3) in the trunk axis direction, and calculating a feature amount showing distribution of air velocity for each of the left lung field region and the right lung field region in the trunk axis direction.

3. The thoracic diagnosis assistance information generation method of claim 1 or 2, wherein calculating the air velocity distribution includes dividing (S27) the lung field region into three or more block regions in a trunk axis direction.

4. The thoracic diagnosis assistance information generation method of any one of claims 1 to 3, wherein calculating (S28) the air velocity distribution includes dividing the lung field region into a plurality of block regions in the trunk axis direction and a direction substantially orthogonal to the trunk axis direction.

5. The thoracic diagnosis assistance information generation method of claim 1, wherein the template is created based on air velocity distribution of a plurality of normal lung fields.

6. A thoracic diagnosis assistance system (100) comprising:

    a capturing section (2) which captures (S4) a dynamic state of a chest portion including at least one breathing cycle to generate a plurality of successive frame images;
    an extracting section (2) which extracts a lung field region from one frame image among the plurality of frame images generated by the capturing section;
    an air velocity calculating section (3) which divides (S23) the lung field region extracted by the extracting section into a plurality of small regions, corresponds the small regions among the plurality of frame images, performs analysis for each corresponded small region, and calculates a feature amount showing air velocity for each small region; and
    an air velocity distribution calculating section (3) which divides (S23) the lung field region into a plurality of block regions in a trunk axis direction, calculates a feature amount showing air velocity of each block region based on a feature amount showing the air velocity of the plurality of small regions included in the divided block regions, and calculates a feature amount showing air velocity distribution of the lung field region in the trunk axis direction based on the calculated feature amount showing the air velocity of each block region, wherein

the air velocity distribution calculating section (3) is configured to read from a storage section a template showing air velocity distribution of a normal lung field and calculate, as a feature amount showing air velocity distribution of the lung field region in the trunk axis direction, a degree of match of air velocity distribution between the lung field region and a lung field region in the template, based on a feature amount showing air velocity of the plurality of block regions and a feature amount showing air velocity in each corresponding region in the template, and

wherein the air velocity distribution calculating section (3) is configured to calculate cross-correlation coefficient (S39) of the lung field region and the lung field region of the template as the degree of match of the air velocity distribution between the lung field region and the lung field region of the template.

7. A dynamic state image processing apparatus (100) comprising:

an extracting section (2) which extracts a lung field region from one frame image among the plurality of frame images obtained by capturing (S4) a dynamic state of a chest portion including at least one breathing cycle;

an air velocity calculating section (3) which divides (S23) the lung field region extracted by the extracting section into a plurality of small regions, corresponds the small regions among the plurality of frame images, performs analysis for each corresponded small region, and calculates a feature amount showing air velocity for each small region; and

an air velocity distribution calculating section (3) which divides (S23) the lung field region into a plurality of block regions in a trunk axis direction, calculates a feature amount showing air velocity of each block region based on a feature amount showing the air velocity of the plurality of small regions included in the divided block regions, and calculates a feature amount showing air velocity distribution of the lung field region in the trunk axis direction based on the calculated feature amount showing the air velocity of each block region, wherein

the air velocity distribution calculating section is configured to read from a storage section a template showing air velocity distribution of a normal lung field and calculate, as a feature amount showing air velocity distribution of the lung field region in the trunk axis direction, a degree of match of air velocity distribution between the lung field region and a lung field region in the template, based on a feature amount showing air velocity of the plurality of block regions and a feature amount showing air velocity in each corresponding region in the template and wherein the air velocity distribution calculating section (3) is configured to calculate cross-correlation coefficient (S39) of the lung field region and the lung field region of the template as the degree of match of the air velocity distribution between the lung field region and the lung field region of the template.

## Patentansprüche

1. Verfahren zur Erzeugung von Thoraxdiagnosehilfsinformationen, umfassend:

Erfassen (S4) eines dynamischen Zustandes eines Brustabschnitts, umfassend zumindest einen Atemzyklus, unter Einsatz eines Detektors (13) mit zweidimensional ausgerichteten Detektionselementen, um eine Vielzahl von aufeinanderfolgenden Einzelbildern zu erzeugen;

Entnehmen (S21) eines Lungenfeldbereichs aus einem Einzelbild aus der Vielzahl von Einzelbildern, die im Rahmen der Erfassung erzeugt worden sind;

Berechnen (S28) der Luftgeschwindigkeit durch Unterteilen des im Rahmen der Entnahme entnommenen Lungenfeldbereichs in eine Vielzahl von kleinen Bereichen, Abgleichen der kleinen Bereiche aus der Vielzahl von Einzelbildern, Durchführen einer Analyse für jeden abgeglichenen kleinen Bereich, und Berechnen einer die Luftgeschwindigkeit jedes kleinen Bereichs zeigenden Merkmalsmenge; und

Berechnen (S28) der Luftgeschwindigkeitsverteilung durch Unterteilen (S23) des Lungenfeldbereichs in eine Vielzahl von Blockbereichen in einer Stammachsenrichtung, Berechnen einer die Luftgeschwindigkeit jedes Blockbereichs zeigenden Merkmalsmenge auf Basis einer die Luftgeschwindigkeit der Vielzahl von kleinen, in den unterteilten Blockbereichen enthaltenen Bereichen zeigenden Merkmalsmenge (S29), und Berechnen einer die Luftgeschwindigkeitsverteilung des Lungenfeldbereichs in der Stammachsenrichtung zeigenden Merkmalsmenge auf Basis der berechneten, die Luftgeschwindigkeit jedes Blockbereichs zeigenden Merkmalsmenge, wobei

das Berechnen der Luftgeschwindigkeitsverteilung das Auslesen eines die Luftgeschwindigkeitsverteilung eines normalen Lungenfelds zeigenden Vorlage aus einer Speichersektion und das Berechnen eines Übereinstimmungsgrads der Luftgeschwindigkeitsverteilung zwischen dem Lungenfeldbereich und einem Lungenfeldbereich der Vorlage als eine die Luftgeschwindigkeitsverteilung des Lungenfeldbereichs in der Stammachsenrichtung zeigende Merkmalsmenge auf Basis einer die Luftgeschwindigkeit der Vielzahl von Blockbereichen zeigenden Merkmalsmenge und einer die Luftgeschwindigkeit in jedem korrespondierenden Bereich der Vorlage zeigenden Merkmalsmenge umfasst, und

wobei das Berechnen (S28) der Luftgeschwindigkeitsverteilung das Berechnen eines Kreuzkorrelationskoeffizienten (S39) des Lungenfeldbereichs und des Lungenfeldbereichs der Vorlage als Übereinstimmungsgrad der Luftgeschwindigkeitsverteilung zwischen dem Lungenfeldbereich und dem Lungenfeldbereich der Vorlage umfasst.

2. Verfahren zur Erzeugung von Thoraxdiagnosehilfsinformationen nach Anspruch 1, wobei das Berechnen (S28) der Luftgeschwindigkeitsverteilung das Unterteilen (S27) sowohl eines linken Lungenfeldbereichs als auch eines rechten Lungenfeldbereichs in eine Vielzahl von Blockbereichen (L1, L2, L3, R1, R2, R3) in der Stammachsenrichtung und das Berechnen einer die Verteilung der Luftgeschwindigkeit zeigenden Merkmalsmenge für sowohl den linken Lungenfeldbereich als auch den rechten Lungenfeldbereich in der Stammachsenrichtung umfasst.

3. Verfahren zur Erzeugung von Thoraxdiagnosehilfsinformationen nach Anspruch 1 oder 2, wobei das Berechnen der Luftgeschwindigkeitsverteilung das Unterteilen (S27) des Lungenfeldbereichs in drei oder mehr Blockbereiche in einer Stammachsenrichtung umfasst.

4. Verfahren zur Erzeugung von Thoraxdiagnosehilfsinformationen nach einem der Ansprüche 1 bis 3, wobei das Berechnen (S28) der Luftgeschwindigkeitsverteilung das Unterteilen des Lungenfeldbereichs in eine Vielzahl von Blockbereichen in der Stammachsenrichtung und in einer zur Stammachsenrichtung im Wesentlichen orthogonalen Richtung umfasst.

5. Verfahren zur Erzeugung von Thoraxdiagnosehilfsinformationen nach Anspruch 1, wobei die Vorlage auf Basis der Luftgeschwindigkeitsverteilung einer Vielzahl von normalen Lungenbereichen erzeugt wird.

6. Thoraxdiagnosehilfssystem (100), umfassend:

eine Erfassungssektion (2), welche (S4) einen dynamischen Zustand eines Brustabschnitts, umfassend zumindest einen Atemzyklus, erfasst, um eine Vielzahl von aufeinanderfolgenden Einzelbildern zu erzeugen;
eine Entnahmesektion (2), welche einen Lungenfeldbereich aus einem Einzelbild aus der Vielzahl von Einzelbildern, die von der Erfassungssektion erzeugt worden sind, entnimmt;
eine Luftgeschwindigkeitsberechnungssektion (3), welche (S23) den von der Entnahmesektion entnommenen Lungenfeldbereich in eine Vielzahl von kleinen Bereichen unterteilt, die kleinen Bereiche aus der Vielzahl von Einzelbildern abgleicht, für jeden abgeglichenen kleinen Bereich eine Analyse durchführt, und eine die Luftgeschwindigkeit jedes kleinen Bereichs zeigende Merkmalsmenge berechnet; und
eine Luftgeschwindigkeitsberechnungssektion (3), welche (S23) den Lungenfeldbereich in eine Vielzahl von Blockbereichen in einer Stammachsenrichtung unterteilt, eine die Luftgeschwindigkeit jedes Blockbereichs zeigende Merkmalsmenge auf Basis einer die Luftgeschwindigkeit der Vielzahl von kleinen, in den unterteilten Blockbereichen enthaltenen Bereichen zeigenden Merkmalsmenge berechnet, und eine die Luftgeschwindigkeitsverteilung des Lungenfeldbereichs in der Stammachsenrichtung zeigenden Merkmalsmenge auf Basis der berechneten, die Luftgeschwindigkeit jedes Blockbereichs zeigenden Merkmalsmenge berechnet, wobei die Luftgeschwindigkeitsberechnungssektion (3) derart konzipiert ist, dass sie eine die Luftgeschwindigkeitsverteilung eines normalen Lungenfelds zeigende Vorlage aus einer Speichersektion ausliest und einen Übereinstimmungsgrad der Luftgeschwindigkeitsverteilung zwischen dem Lungenfeldbereich und einem Lungenfeldbereich der Vorlage als eine die Luftgeschwindigkeitsverteilung des Lungenfeldbereichs in der Stammachsenrichtung zeigende Merkmalsmenge auf Basis einer die Luftgeschwindigkeit der Vielzahl von Blockbereichen zeigenden Merkmalsmenge und einer die Luftgeschwindigkeit in jedem korrespondierenden Bereich der Vorlage zeigenden Merkmalsmenge berechnet, und
wobei die Luftgeschwindigkeitsberechnungssektion (3) derart konzipiert ist, dass sie einen Kreuzkorrelationskoeffizienten (S39) des Lungenfeldbereichs und des Lungenfeldbereichs der Vorlage als Übereinstimmungsgrad der Luftgeschwindigkeitsverteilung zwischen dem Lungenfeldbereich und dem Lungenfeldbereich der Vorlage berechnet.

7. Vorrichtung zur Verarbeitung eines dynamischen Zustandsbilds (100), umfassend:

eine Entnahmesektion (2), welche einen Lungenfeldbereich aus einem Einzelbild aus der Vielzahl von Einzelbildern, die durch Erfassen (S4) eines dynamischen Zustands eines Brustabschnitts, umfassend zumindest einen Atemzyklus, erhalten worden sind, entnimmt;

eine Luftgeschwindigkeitsberechnungssektion (3), welche (S23) den von der Entnahmesektion entnommenen Lungenfeldbereich in eine Vielzahl von kleinen Bereichen unterteilt, die kleinen Bereiche aus der Vielzahl von Einzelbildern abgleicht, für jeden abgeglichenen kleinen Bereich eine Analyse durchführt, und eine die Luftgeschwindigkeit jedes kleinen Bereichs zeigende Merkmalsmenge berechnet; und

eine Luftgeschwindigkeitsberechnungssektion (3), welche (S23) den Lungenfeldbereich in eine Vielzahl von Blockbereichen in einer Stammachsenrichtung unterteilt, eine die Luftgeschwindigkeit jedes Blockbereichs zeigende Merkmalsmenge auf Basis einer die Luftgeschwindigkeit der Vielzahl von kleinen, in den unterteilten Blockbereichen enthaltenen Bereichen zeigenden Merkmalsmenge berechnet, und eine die Luftgeschwindigkeitsverteilung des Lungenfeldbereichs in der Stammachsenrichtung zeigenden Merkmalsmenge auf Basis der berechneten, die Luftgeschwindigkeit jedes Blockbereichs zeigenden Merkmalsmenge berechnet, wobei die Luftgeschwindigkeitsberechnungssektion derart konzipiert ist, dass sie eine die Luftgeschwindigkeitsverteilung eines normalen Lungenfelds zeigende Vorlage aus einer Speichersektion ausliest und einen Übereinstimmungsgrad der Luftgeschwindigkeitsverteilung zwischen dem Lungenfeldbereich und einem Lungenfeldbereich der Vorlage als eine die Luftgeschwindigkeitsverteilung des Lungenfeldbereichs in der Stammachsenrichtung zeigende Merkmalsmenge auf Basis einer die Luftgeschwindigkeit der Vielzahl von Blockbereichen zeigenden Merkmalsmenge und einer die Luftgeschwindigkeit in jedem korrespondierenden Bereich der Vorlage zeigenden Merkmalsmenge berechnet, und

wobei die Luftgeschwindigkeitsberechnungssektion (3) derart konzipiert ist, dass sie einen Kreuzkorrelationskoeffizienten (S39) des Lungenfeldbereichs und des Lungenfeldbereichs der Vorlage als Übereinstimmungsgrad der Luftgeschwindigkeitsverteilung zwischen dem Lungenfeldbereich und dem Lungenfeldbereich der Vorlage berechnet.

## Revendications

1. Un procédé de génération d'informations pour assistance au diagnostic thoracique comprenant les étapes consistant en:

- la capture (S4) d'un état dynamique d'une partie de torse, incluant au moins un cycle de respiration, par utilisation d'un détecteur (13) pourvu d'éléments de détection alignés bi-dimensionnellement pour générer une pluralité d'images de trames successives;
- l'extraction (S21) d'une région du champ pulmonaire à partir d'une image de trames de la pluralité d'images de trames générées lors de la capture;
- le calcul (S28) de la vitesse de l'air en divisant la région du champ pulmonaire, extraite lors de l'extraction en une pluralité de petites régions, en faisant correspondre les petites régions de la pluralité d'images de trames, en effectuant l'analyse pour chaque petite région correspondante et en calculant une quantité caractéristique indiquant la vitesse de l'air de chaque petite région; et
- le calcul (S28) de la répartition de la vitesse de l'air par division (S23) de la région du champ pulmonaire en une pluralité de régions de blocs selon la direction de l'axe du tronc, par calcul d'une quantité caractéristique indiquant la vitesse de l'air pour chaque région de bloc sur la base d'une quantité caractéristique montrant (S29) la vitesse de l'air de la pluralité des petites régions incluses dans les régions de blocs divisées, et par calcul d'une quantité caractéristique montrant la répartition de la vitesse de l'air de la région du champ pulmonaire selon la direction de l'axe du tronc sur la base de la quantité caractéristique calculée indiquant la vitesse de l'air de chaque région de blocs,

procédé dans lequel

▪ le calcul de la répartition de la vitesse de l'air comprend la lecture d'une section de stockage d'un gabarit montrant la répartition de la vitesse de l'air d'un champ pulmonaire normal et le calcul comme quantité caractéristique montrant la répartition de la vitesse de l'air de la région du champ pulmonaire selon la direction de l'axe de tronc, comprend un degré de correspondance de la répartition de la vitesse de l'air entre la région du champ pulmonaire et la région du champ pulmonaire sur le gabarit, comprend, basé sur une quantité caractéristique indiquant la vitesse de l'air de la pluralité des régions de blocs et une quantité caractéristique indiquant la vitesse de l'air dans chaque région correspondante du gabarit, et
▪ le calcul (S28) de la répartition de la vitesse de l'air comprend le calcul du coefficient de corrélation croisée (S39) de la région du champ pulmonaire et de la région du champ pulmonaire du gabarit comme taux de correspondance de la répartition de la vitesse de l'air entre la région du champ pulmonaire et la région du champ pulmonaire du gabarit.

2. Le procédé de génération d'informations pour assistance au diagnostic thoracique selon la revendication 1, dans lequel le calcul (S28) de la répartition de la vitesse de l'air inclut la division (S27) de chacune des régions de champs pulmonaires gauches et droites en une pluralité de régions de blocs (L1, L2, L3, R1, R2, R3) selon la direction de l'axe du tronc et le calcul de la quantité caractéristique montrant la répartition de la vitesse de l'air pour chacune des régions du champ pulmonaire gauche et du champ pulmonaire droit selon la direction de l'axe du tronc.

3. Le procédé de génération d'informations pour assistance au diagnostic thoracique selon la revendication 1 ou 2, dans lequel le calcul de la répartition de la vitesse de l'air inclut la division (S27) de la région du champ pulmonaire en trois ou davantage de régions de blocs selon la direction de l'axe du tronc.

4. Le procédé de génération d'informations pour assistance au diagnostic thoracique selon l'une quelconque des revendications 1 à 3, dans lequel le calcul (S28) de la répartition de la vitesse de l'air inclut la division de la région du champ pulmonaire en une pluralité de régions de blocs selon la direction de l'axe du tronc et la direction sensiblement orthogonale à la direction de l'axe du tronc.

5. Le procédé de génération d'informations pour assistance au diagnostic thoracique selon la revendication 1, dans lequel le gabarit est créé sur la base de la répartition de la vitesse de l'air d'une pluralité de champs pulmonaires normaux.

6. Un système d'assistance de diagnostic thoracique (100) comprenant:

   - une section de capture (2) qui capture (S4) un état dynamique d'une partie de torse incluant au moins un cycle de respiration pour générer une pluralité d'images de trames successives;
   - une section d'extraction (2) qui extrait une région du champ pulmonaire d'une image de trames de la pluralité d'images de trames générées par la section de capture;
   - une section de calcul de vitesse de l'air (3) qui divise (S23) la région du champ pulmonaire extraite par la section d'extraction en une pluralité de petites régions, fait correspondre les petites régions parmi la pluralité d'images de trame, qui effectue l'analyse pour chaque petite région correspondante et qui calcule une quantité caractéristique pour chaque petite région; et
   - une section de calcul de la répartition de la vitesse de l'air (3) qui divise (S23) la région du champ pulmonaire en une pluralité de régions de blocs selon la direction de l'axe du tronc, qui calcule une quantité caractéristique indiquant la vitesse de l'air de chaque région de blocs sur la base d'une quantité caractéristique indiquant la vitesse de l'air de la pluralité de petites régions incluses dans les régions de blocs divisés et qui calcule une quantité caractéristique montrant la répartition de la vitesse de l'air de la région du champ pulmonaire selon la direction de l'axe du tronc sur la base de la quantité caractéristique calculée indiquant la vitesse de l'air de chaque région de blocs,

   système dans lequel:

   ▪ une section de calcul de la répartition de la vitesse de l'air (3) est configurée pour la lecture, depuis une section de stockage, un gabarit montrant la répartition de la vitesse d'un champ pulmonaire normal et calcule, comme quantité caractéristique montrant la répartition de la vitesse de l'air de la région du champ pulmonaire selon la direction de l'axe du tronc, un taux de correspondance de la répartition de la vitesse de l'air entre la région du champ pulmonaire et la région du champ pulmonaire sur le gabarit, basé sur une quantité caractéristique indiquant la vitesse de l'air de la pluralité des régions de blocs et une quantité caractéristique indiquant la vitesse de l'air dans chaque région correspondante du gabarit, et
   ▪ la section de calcul de la répartition de la vitesse de l'air (3) est configurée pour calculer le coefficient de corrélation croisée (S39) de la région du champ pulmonaire et de la région du champ pulmonaire du gabarit comme degré de correspondance de la répartition de la vitesse de l'air entre la région du champ pulmonaire et la région du champ pulmonaire du gabarit.

7. Un appareil de traitement dynamique d'image (100) comprenant:

   - une section d'extraction (2) qui extrait une région de champ pulmonaire d'une image de trames parmi la pluralité d'images de trames obtenues par capture (S4) d'un état dynamique d'une partie de torse incluant au moins un cycle de respiration;

- une section de calcul de vitesse de l'air (3) qui divise (S23) la région du champ pulmonaire extraite par la section d'extraction en une pluralité de petites régions, qui correspond aux petites régions de la pluralité d'images de trames, qui effectue l'analyse pour chaque petite région correspondante et qui calcule une quantité caractéristique indiquant la vitesse de l'air de chaque petite région; et

- une section de calcul de la répartition de la vitesse de l'air (3) par division (S26) de la région du champ pulmonaire en une pluralité de régions de blocs selon la direction de l'axe du tronc, par calcul d'une quantité caractéristique indiquant la vitesse de l'air pour chaque région de blocs sur la base d'une quantité caractéristique montrant la vitesse de l'air de la pluralité de petites régions incluses dans les régions de blocs divisés, et par calcul d'une quantité caractéristique montrant la répartition de la vitesse de l'air de la région du champ pulmonaire selon la direction de l'axe du tronc sur la base de la quantité caractéristique calculée indiquant la vitesse de l'air de chaque région de blocs,

appareil dans lequel

- la section de calcul de la répartition de la vitesse de l'air est configurée afin de lecture d'une section de stockage, un gabarit montrant la répartition de la vitesse de l'air d'un champ pulmonaire normal et de calculer, comme quantité caractéristique montrant la répartition de la vitesse de l'air de la région du champ pulmonaire selon la direction de l'axe de tronc, de déterminer un taux de correspondance de la répartition de la vitesse de l'air entre la région du champ pulmonaire et la région du champ pulmonaire sur le gabarit, basé sur une quantité caractéristique indiquant la vitesse de l'air de la pluralité des régions de blocs et une quantité caractéristique indiquant la vitesse de l'air dans chaque région correspondante du gabarit, et

- la section de calcul de la répartition de la vitesse de l'air (3) est configurée pour calculer le coefficient de corrélation croisée (S39) de la région du champ pulmonaire et de la région du champ pulmonaire du gabarit comme degré de correspondance de la répartition de la vitesse de l'air entre la région du champ pulmonaire et la région du champ pulmonaire du gabarit.

# FIG.1

100

DIAGNOSIS CONSOLE 3
- CONTROL SECTION 31
- STORAGE SECTION 32
- OPERATION SECTION 33
- DISPLAY SECTION 34
- COMMUNICATION SECTION 35
- 36

CAPTURING CONSOLE 2
- CONTROL SECTION 21
- STORAGE SECTION 22
- OPERATION SECTION 23
- DISPLAY SECTION 24
- COMMUNICATION SECTION 25
- 26

NT

1

M
13 14

IMAGE READING CONDITION
READING CONTROL APPARATUS
IMAGE DATA

SYNCHRONIZING SIGNAL

RADIATION EMITTING CONDITION

11

12
RADIATION EMITTING CONTROL APPARATUS

# FIG.2

```
          ( START )
              │
              ▼
  ┌──────────────────────────┐
  │ INPUT PATIENT INFORMATION │──── S1
  └──────────────────────────┘
              │
              ▼
  ┌────────────────────────────────┐
  │ SET RADIATION EMITTING CONDITION, │──── S2
  │    IMAGE READING CONDITION      │
  └────────────────────────────────┘
              │
        ┌─────▼──────
        │      ╱╲        S3
   NO   │    ╱ IS THERE  ╲
  ◄─────┤   ╱ RADIATION   ╲
        │   ╲ EMITTING    ╱
        │    ╲INSTRUCTION?╱
        │      ╲╱
              │ YES
              ▼
  ┌──────────────────────────┐
  │   CAPTURE DYNAMIC STATE   │──── S4
  └──────────────────────────┘
              │
              ▼
  ┌──────────────────────────┐
  │ PERFORM CORRECTION PROCESSING │──── S5
  └──────────────────────────┘
              │
              ▼
  ┌──────────────────────────┐
  │  STORE DYNAMIC STATE IMAGE │──── S6
  └──────────────────────────┘
              │
              ▼
  ┌──────────────────────────┐
  │ DISPLAY DYNAMIC STATE IMAGE │──── S7
  └──────────────────────────┘
              │
              ▼          S8
            ╱╲              NO
          ╱ OK? ╲──────────────────┐
           ╲    ╱                   │
             ╲╱                     ▼
           │ YES    S9        ┌──────────────────────────┐ S10
           ▼                  │ DELETE DYNAMIC STATE IMAGE │
  ┌──────────────────────────┐└──────────────────────────┘
  │ TRANSMIT DYNAMIC STATE IMAGE │        │
  │   TO DIAGNOSIS CONSOLE    │◄──────────┘
  └──────────────────────────┘
              │
              ▼
          ( END )
```

# FIG.3

( IMAGE ANALYSIS PROCESSING )

EXTRACT LUNG FIELD REGION — *S21*

PERFORM LOW PASS FILTERING PROCESSING — *S22*

DIVIDE LUNG FIELD REGIONS INTO SMALL REGIONS — *S23*

PERFORM FRAME DIFFERENCE
PROCESSING IN EACH SMALL REGION — *S24*

CLASSIFY TO FRAME IMAGE GROUP OF EXPIRATION PERIOD
AND FRAME IMAGE GROUP OF INSPIRATION PERIOD — *S25*

MAKE MAXIMUM AIR VELOCITY IMAGE FOR EACH OF
EXPIRATION PERIOD AND INSPIRATION PERIOD — *S26*

DIVIDE LUNG FIELD REGION OF MAXIMUM AIR
VELOCITY IMAGE INTO PLURALITY OF BLOCK REGIONS,
AND CALCULATE AVERAGE VALUE OF MAXIMUM
AIR VELOCITY FOR EACH BLOCK REGION — *S27*

CALCULATE DIFFERENCE VALUE OF MAXIMUM AIR VELOCITY
AVERAGE VALUE AMONG ADJACENT BLOCK REGIONS
IN TRUNK AXIS DIRECTION
AND CALCULATE FEATURE AMOUNT SHOWING MAXIMUM
AIR VELOCITY DISTRIBUTION BASED ON ABOVE — *S28*

DISPLAY ANALYSIS RESULT — *S29*

( END )

# FIG.4

T = t₀

T = t₁

T = t₂

MAXIMUM INSPIRATION LEVEL

EXPIRATION PERIOD

T = t₃

T = t₄

T = t₅

MAXIMUM EXPIRATION LEVEL

INSPIRATION PERIOD

T = t₆

MAXIMUM INSPIRATION LEVEL

# FIG.5

RESTING EXPIRATION LEVEL,
RESTING BREATHING

RESTING INSPIRATION LEVEL,
RESTING BREATHING

*FIG.6*

D4

D3

A31

A4

A3

RESTING INSPIRATION LEVEL, RESTING BREATHING

RESTING EXPIRATION LEVEL, RESTING BREATHING

## FIG.7A

R3  R2  R1  L3  L2  L1

## FIG.7B

# FIG.7C

# FIG.8A

MAXIMUM AIR VELOCITY DISTRIBUTION FEATURE AMOUNT

| DATA NAME | INSPIRATION_ RIGHT LUNG FIELD | INSPIRATION_ LEFT LUNG FIELD | EXPIRATION_ RIGHT LUNG FIELD | EXPIRATION_ LEFT LUNG FIELD |
|---|---|---|---|---|
| NORMAL | 1 | 1 | 1 | 1 |

MAXIMUM AIR VELOCITY IMAGE

INSPIRATION AIR VELOCITY

SLOW ▭ FAST

←——AIR VELOCITY——→

EXPIRATION AIR VELOCITY

SLOW ▭ FAST

←——AIR VELOCITY——→

# *FIG.8B*

### MAXIMUM AIR VELOCITY DISTRIBUTION FEATURE AMOUNT

| DATA NAME | INSPIRATION_ RIGHT LUNG FIELD | INSPIRATION_ LEFT LUNG FIELD | EXPIRATION_ RIGHT LUNG FIELD | EXPIRATION_ LEFT LUNG FIELD |
|---|---|---|---|---|
| NORMAL | 1 | 1 | 1 | 1 |

### MAXIMUM AIR VELOCITY IMAGE

INSPIRATION AIR VELOCITY

EXPIRATION AIR VELOCITY

SLOW ← AIR VELOCITY → FAST       SLOW ← AIR VELOCITY → FAST

# FIG.8C

## MAXIMUM AIR VELOCITY DISTRIBUTION FEATURE AMOUNT

| DATA NAME | INSPIRATION RIGHT LUNG FIELD | INSPIRATION LEFT LUNG FIELD | EXPIRATION RIGHT LUNG FIELD | EXPIRATION LEFT LUNG FIELD |
|---|---|---|---|---|
| NORMAL | 1 | 1 | 1 | 1 |

### DISPLAY OF BLOCK REGION

### INSPIRATION MAXIMUM AIR VELOCITY AVERAGE VALUE

[1] a
[2] b
[3] c
[4] d
[5] e
[6] f

### EXPIRATION MAXIMUM AIR VELOCITY AVERAGE VALUE

[1] g
[2] h
[3] i
[4] j
[5] k
[6] l

EP 2 674 912 B1

# *FIG.9*

```
( IMAGE ANALYSIS PROCESSING B )
              |
              v
┌──────────────────────────────────┐
│     EXTRACT LUNG FIELD REGION     │── S31
└──────────────────────────────────┘
              |
              v
┌──────────────────────────────────┐
│ PERFORM LOW PASS FILTERING PROCESSING │── S32
└──────────────────────────────────┘
              |
              v
┌──────────────────────────────────┐
│ DIVIDE LUNG FIELD REGION INTO SMALL REGIONS │── S33
└──────────────────────────────────┘
              |
              v
┌──────────────────────────────────┐
│ PERFORM FRAME DIFFERENCE PROCESSING │── S34
│        IN EACH SMALL REGION        │
└──────────────────────────────────┘
              |
              v
┌──────────────────────────────────┐
│ CLASSIFY TO FRAME IMAGE GROUP OF EXPIRATION PERIOD │── S35
│ AND FRAME IMAGE GROUP OF INSPIRATION PERIOD │
└──────────────────────────────────┘
              |
              v
┌──────────────────────────────────┐
│     MAKE MAXIMUM AIR VELOCITY IMAGE FOR     │── S36
│ EACH OF EXPIRATION PERIOD AND INSPIRATION PERIOD │
└──────────────────────────────────┘
              |
              v
┌──────────────────────────────────┐
│ DIVIDE LUNG FIELD REGION OF MAXIMUM AIR VELOCITY IMAGE │── S37
│        INTO PLURALITY OF BLOCK REGIONS,        │
│ AND CALCULATE AVERAGE VALUE OF MAXIMUM AIR VELOCITY │
│           FOR EACH BLOCK REGION           │
└──────────────────────────────────┘
              |
              v
┌──────────────────────────────────┐
│ READ NORMAL AIR VELOCITY DISTRIBUTION TEMPLATE │── S38
│            FROM STORAGE SECTION            │
└──────────────────────────────────┘
              |
              v
┌──────────────────────────────────┐
│ CALCULATE CROSS-CORRELATION VALUE OF LUNG FIELD REGION │── S39
│ OF MAXIMUM AIR VELOCITY IMAGE AND LUNG FIELD REGION │
│ OF NORMAL AIR VELOCITY DISTRIBUTION TEMPLATE │
└──────────────────────────────────┘
              |
              v
┌──────────────────────────────────┐
│       DISPLAY ANALYSIS RESULT      │── S40
└──────────────────────────────────┘
              |
              v
          (   END   )
```

34

## FIG.10A

## FIG.10B

# FIG.11

*34a*     *34b*     *34c*

SLOW ▬ FAST   SLOW ▬ FAST   SMALL ▬ LARGE

←AIR VELOCITY→   ←AIR VELOCITY→   ←AIR VELOCITY→ RATIO

No.6666 RESTING VENTILATION MAXIMUM AIR VELOCITY RATIO   *34d*
BLOCK SIZE 2mm

AVERAGE VALUE: -0.03
STANDARD DEVIATION: 0.17

PERCENTAGE OF DATA

MAXIMUM AIR VELOCITY RATIO, LOGARITHMIC VALUE OF TPFI/TPFE

SMALL ▬ LARGE

←————AIR VELOCITY RATIO————→

36

# FIG.12

| 34a | 34b | 34c |

SLOW ▬▬▬ FAST   SLOW ▬▬▬ FAST   SMALL ▬▬▬ LARGE

←AIR VELOCITY→   ←AIR VELOCITY→   ←AIR VELOCITY→
RATIO

No.0012 RESTING VENTILATION MAXIMUM AIR VELOCITY RATIO   *34d*
BLOCK SIZE 2mm

AVERAGE VALUE: 0.17
STANDARD DEVIATION: 0.20

PERCENTAGE OF DATA

20%
18%
16%
14%
12%
10%
8%
6%
4%
2%
0%

-1.5  -1.2  -0.9  -0.6  -0.3  0  0.3  0.6  0.9  1.2  1.5

MAXIMUM AIR VELOCITY RATIO, LOGARITHMIC VALUE OF TPFI/TPFE

SMALL ▬▬▬▬▬▬▬ LARGE

←————————AIR VELOCITY RATIO————————→

# FIG.13

*34a*

*34b*

*34c*

SLOW ▦▦▦ FAST   SLOW ▦▦▦ FAST   SMALL ▦▦▦ LARGE

←AIR VELOCITY→   ←AIR VELOCITY→   ←AIR VELOCITY→
RATIO

No.0025 RESTING VENTILATION MAXIMUM AIR VELOCITY RATIO
BLOCK SIZE 2mm

*34d*

AVERAGE VALUE: 0.47
STANDARD DEVIATION: 0.31

PERCENTAGE OF DATA

20%
18%
16%
14%
12%
10%
8%
6%
4%
2%
0%

-1.5  -1.2  -0.9  -0.6  -0.3  0  0.3  0.6  0.9  1.2  1.5

MAXIMUM AIR VELOCITY RATIO, LOGARITHMIC VALUE OF TPFI/TPFE

SMALL ▦▦▦▦▦▦▦ LARGE

←————————AIR VELOCITY RATIO————————→

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

•   WO 2012026146 A

**Non-patent literature cited in the description**

•   **MIKIO TAKAGI ; HARUHISA SHIMODA.** New Edition Image Analysis Handbook. University of Tokyo Press, 2004

•   **KIYOSHI ICHIHARA.** Statistics in Bio-Science. NANKODO, 1990